# EUROPEAN PATENT APPLICATION

(11) **EP 4 792 727 A1**
(43) Date of publication of application: **19.08.2026**
(21) Application number: 24877132.1
(22) Date of filing: 04.10.2024
(51) Int. Cl.: A61B 1/018, A61C 19/04, H04N 23/56, H04N 23/60, H04N 23/63

(54) **IMAGE PROCESSING METHOD, IMAGE PROCESSING SYSTEM, AND PROGRAM**

(30) Priority: 13.10.2023 JP 2023177750
(71) Applicant: Panasonic Intellectual Property Management Co., Ltd., Kadoma-shi, Osaka 571-0057 (JP)
(72) Inventor: HAMASAKI, Takeshi, Kadoma-shi, Osaka 571-0057 (JP); OHTSUKA, Yoshio, Kadoma-shi, Osaka 571-0057 (JP); KITAHARA, Suguru, Takatsuki-shi, Osaka 569-1194 (JP); YAMASHINA, Hiroyuki, Takatsuki-shi, Osaka 569-1194 (JP)
(74) Representative: Novagraaf International SA
(86) International application number: PCT/JP2024/035690
(87) International publication number: WO 2025/079536

(57) **Abstract**

An image processing method is for displaying a dental plaque area on the basis of an RGB image obtained by capturing an image of a tooth and dental plaque fluorescing in response to exposure to light including a blue-light wavelength range. The image processing method includes obtaining the incident level of external light entering the imaging area of the RGB image (S12), performing dental plaque detection processing on the basis of the RGB image (S13), and changing the display mode of a dental plaque detection result according to the incident level of the external light which has entered the imaging area of the RGB image (S15, S16).

## Description

### [Technical Field]

The present disclosure relates to an image processing method, an image processing system, and a program.

### [Background Art]

Patent Literature (PTL) 1 discloses an image processing apparatus that performs color correction on an image captured in a state in which light was not blocked.

### [Citation List]

### [Patent Literature]

[PTL 1] Japanese Unexamined Patent Application Publication No. 2008-244794

### [Summary of Invention]

### [Technical Problem]

Incidentally, detection of a dental plaque area inside an oral cavity by capturing the oral cavity with an imaging apparatus is considered. In this case, a dental plaque area may be detected by error in detecting, for example, dental plaque inside the oral cavity using the imaging apparatus in a state in which external light is entering the oral cavity.

Here, the present disclosure provides an image processing method, an image processing system, and a program that are capable of notifying that a dental plaque area may have been detected by error due to the external light that entered an oral cavity.

### [Solution to Problem]

An image processing method according to one aspect of the present disclosure is an image processing method for displaying a dental plaque area based on an RGB image obtained by capturing an image of a tooth and dental plaque fluorescing in response to exposure to light including a blue-light wavelength range, the image processing method including: obtaining the incident level of external light entering an imaging area of the RGB image; performing dental plaque detection processing based on the RGB image; and changing the display mode of a dental plaque detection result according to the incident level of the external light which has entered the imaging area of the RGB image.

An image processing system according to another aspect of the present disclosure is an image processing system for displaying a dental plaque area based on an RGB image obtained by capturing an image of a tooth and dental plaque fluorescing in response to exposure to light including a blue-light wavelength range, the image processing system including: an obtainer that obtains the incident level of external light entering an imaging area of the RGB image; a dental plaque detector that performs dental plaque detection processing based on the RGB image; and a display controller that changes the display mode of a dental plaque detection result according to the incident level of the external light which has entered the imaging area of the RGB image.

A program according to still another aspect of the present disclosure is a program for causing a computer to execute the above image processing method.

### [Advantageous Effects of Invention]

One aspect of the present disclosure can achieve, for example, an image processing method by which it is possible to notify that a dental plaque area may have been detected by error due to the external light that entered the oral cavity.

### [Brief Description of Drawings]

[FIG. 1]

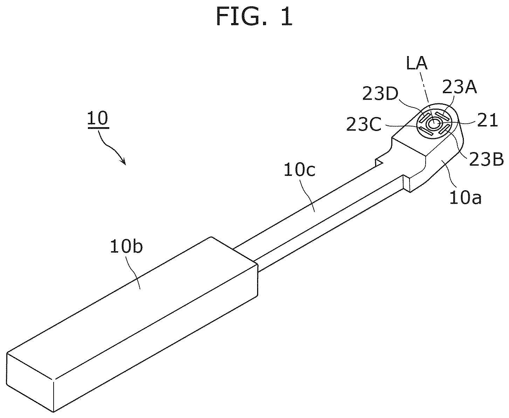

   FIG. 1 is a perspective view of the intraoral camera of an intraoral camera system according to an embodiment.
[FIG. 2]

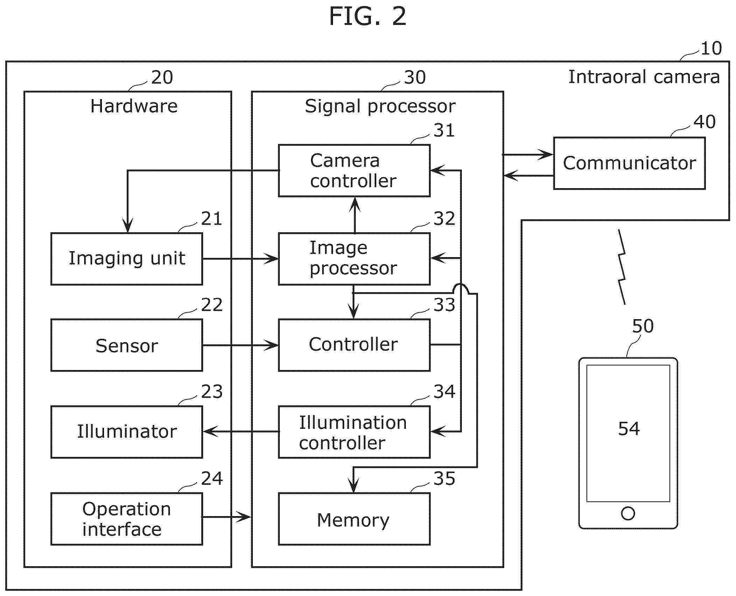

   FIG. 2 illustrates a schematic configuration of the intraoral camera system according to the embodiment.
[FIG. 3]

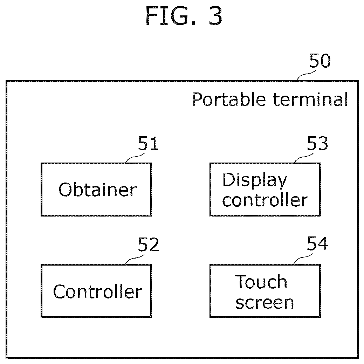

   FIG. 3 is a block diagram illustrating a functional configuration of a portable terminal according to the embodiment.
[FIG. 4]

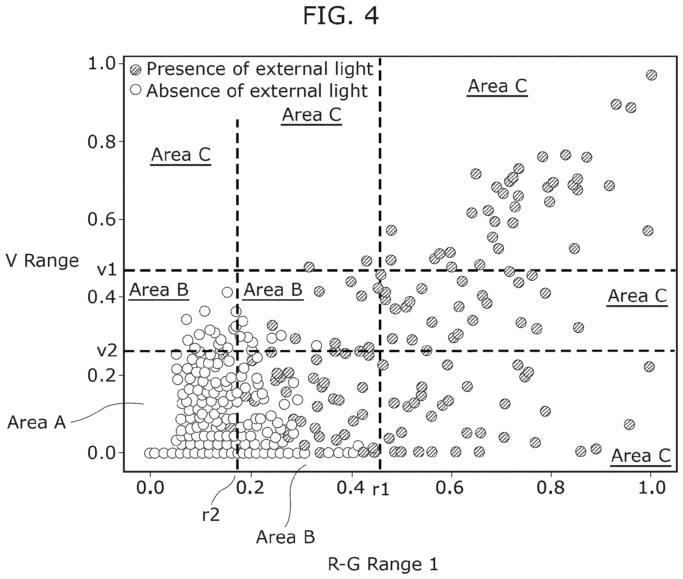

   FIG. 4 is a first figure with the plotted results of determination as to whether external light is present according to the embodiment.
[FIG. 5]

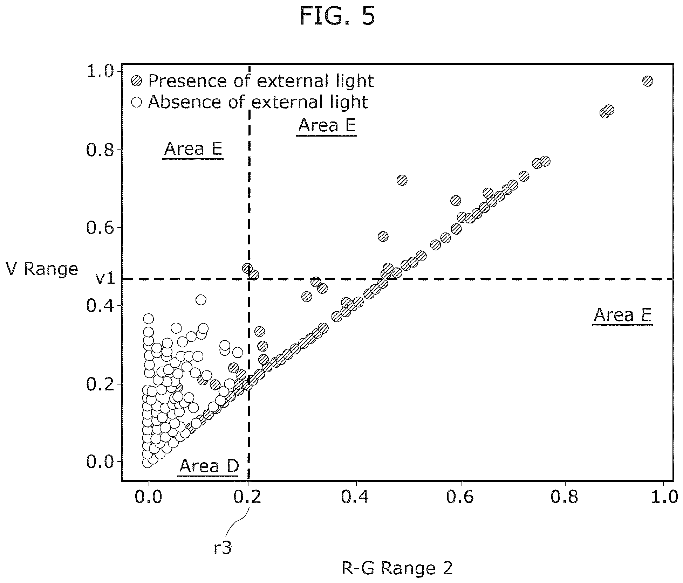

   FIG. 5 is a second figure with the plotted results of determination as to whether external light is present according to the embodiment.
[FIG. 6]

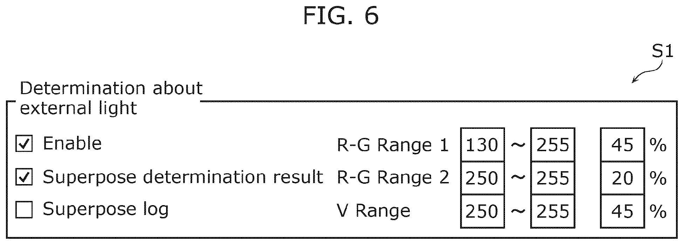

   FIG. 6 illustrates a setting screen of determination about external light according to the embodiment.
[FIG. 7]

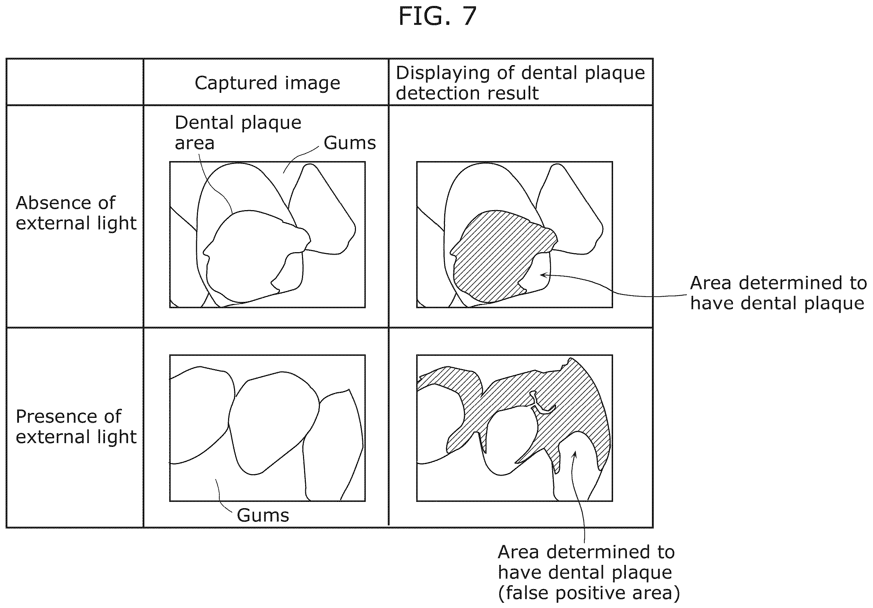

   FIG. 7 illustrates captured images and display examples of dental plaque detection results for cases with and without the presence of external light according to the embodiment.
[FIG. 8]

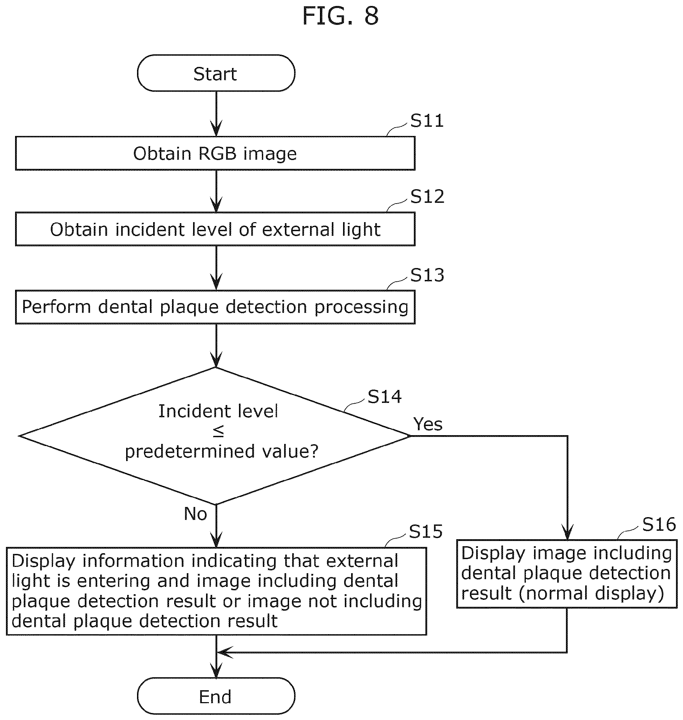

   FIG. 8 is a flowchart illustrating an operation of the portable terminal according to the embodiment.
[FIG. 9]

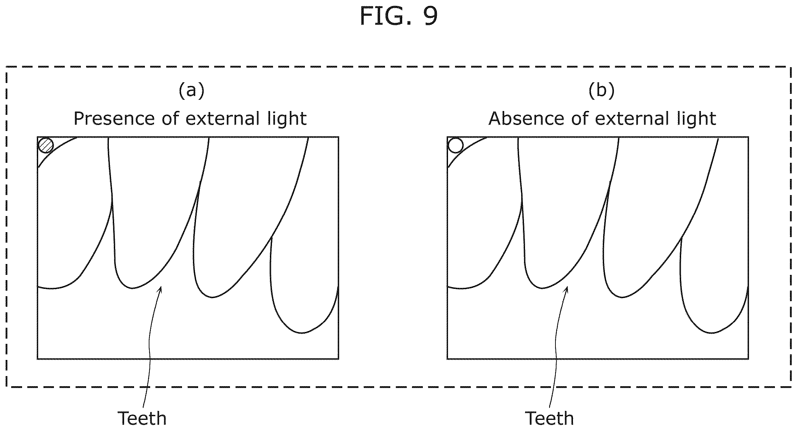

   FIG. 9 illustrates display examples indicating the presence and absence of external light according to the embodiment.
[FIG. 10]

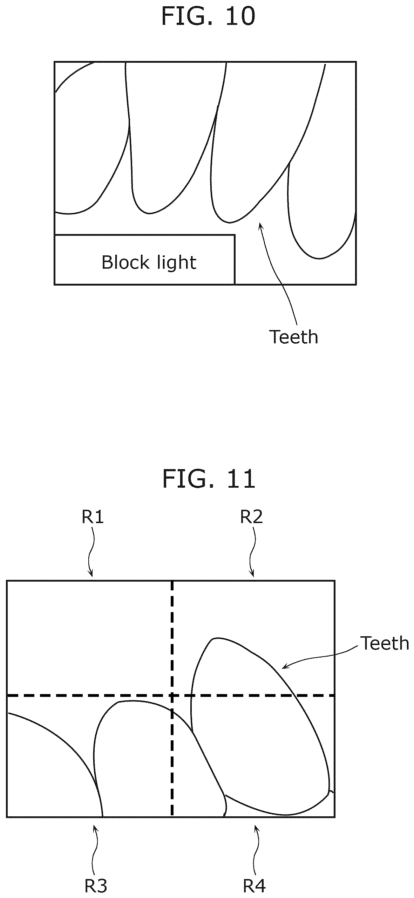

   FIG. 10 illustrates an example of displayed information suggesting blocking light according to the embodiment.
[FIG. 11]
   FIG. 11 schematically illustrates a state where a captured image is divided into a plurality of areas according to the Variation 1 of the embodiment.
[FIG. 12]

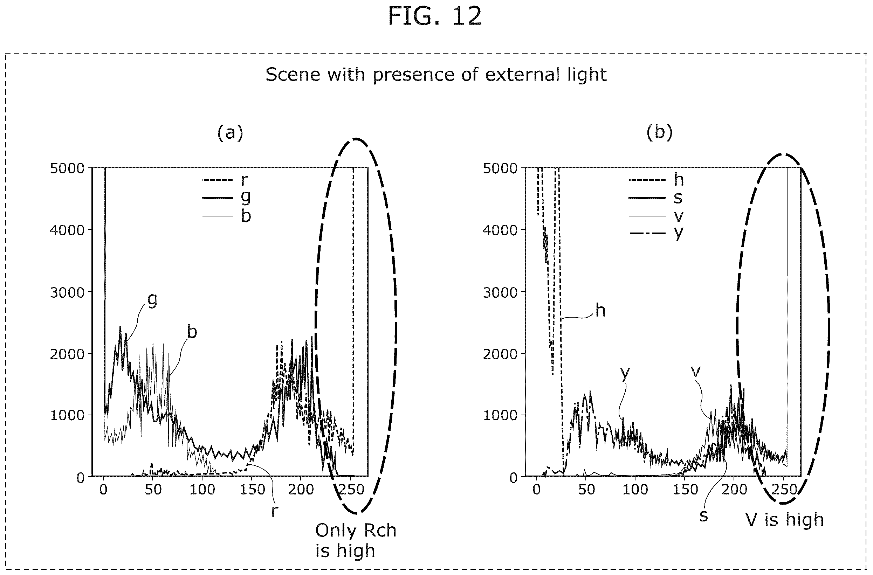

   FIG. 12 illustrates a relationship between values based on respective pixel values and the number of pixels in a scene with the presence of external light.
[FIG. 13]

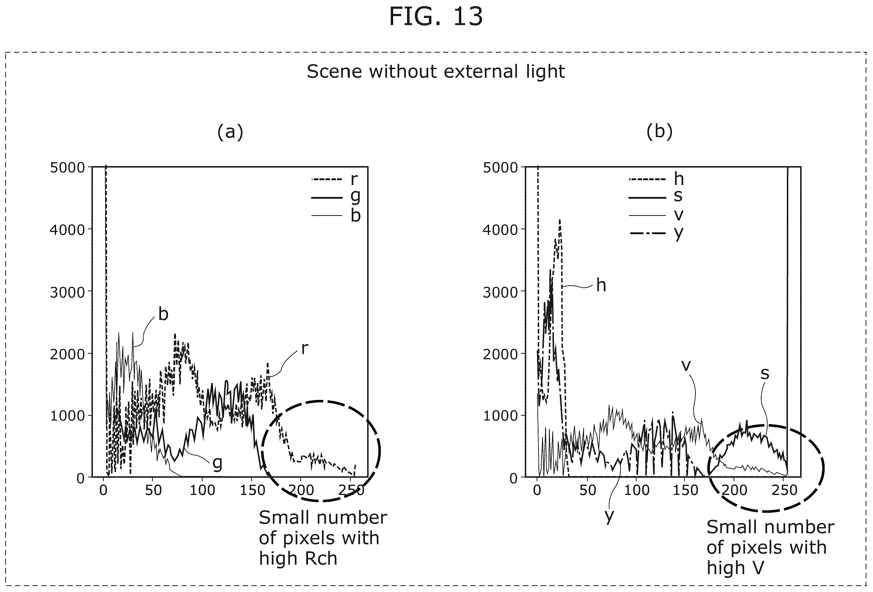

   FIG. 13 illustrates a relationship between values based on respective pixel values and the number of pixels in a scene without external light.
[FIG. 14]

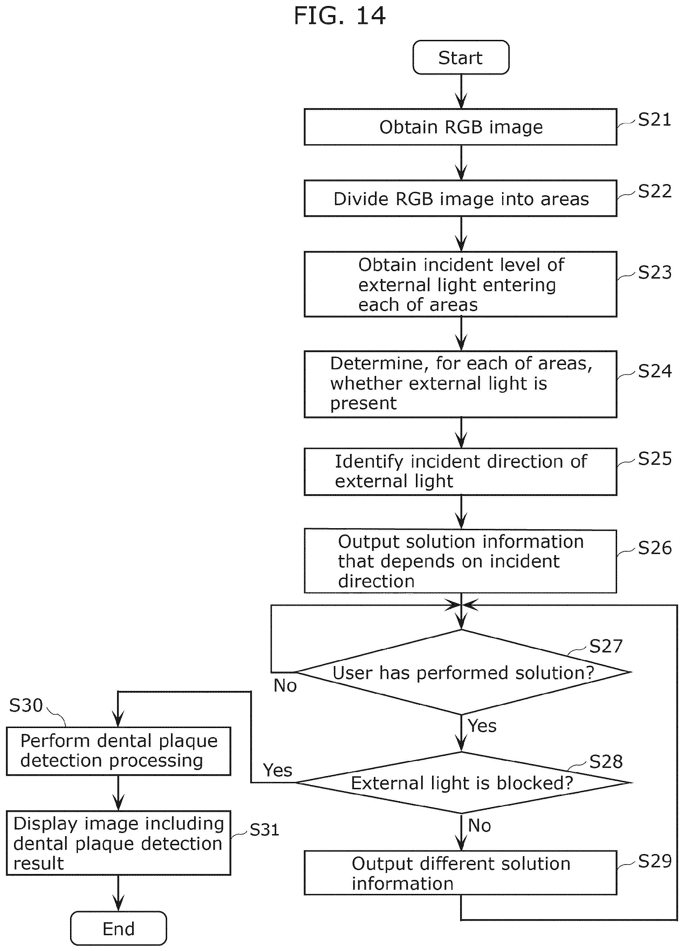

   FIG. 14 is a flowchart illustrating an operation of the portable terminal according to Variation 2 of the embodiment.
[FIG. 15A]

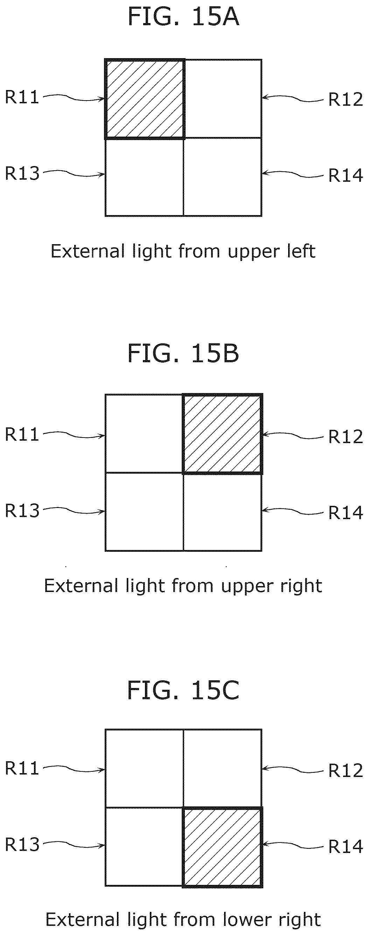

   FIG. 15A illustrates an example of the determination results of incident levels in the four divided areas when external light enters from the upper left.
[FIG. 15B]
   FIG. 15B illustrates an example of the determination results of incident levels in the four divided areas when external light enters from the upper right.
[FIG. 15C]
   FIG. 15C illustrates an example of the determination results of incident levels in the four divided areas when external light enters from the lower right.
[FIG. 15D]

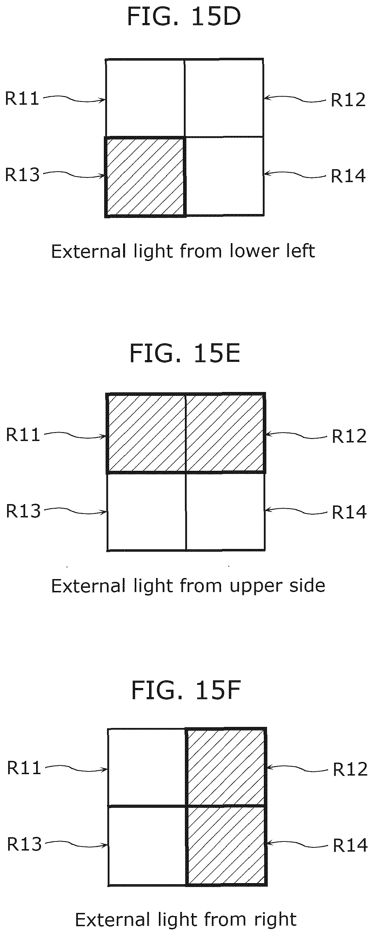

   FIG. 15D illustrates an example of the determination results of incident levels in the four divided areas when external light enters from the lower left.
[FIG. 15E]
   FIG. 15E illustrates an example of the determination results of incident levels in the four divided areas when external light enters from the upper side.
[FIG. 15F]
   FIG. 15F illustrates an example of the determination results of incident levels in the four divided areas when external light enters from the right.
[FIG. 15G]

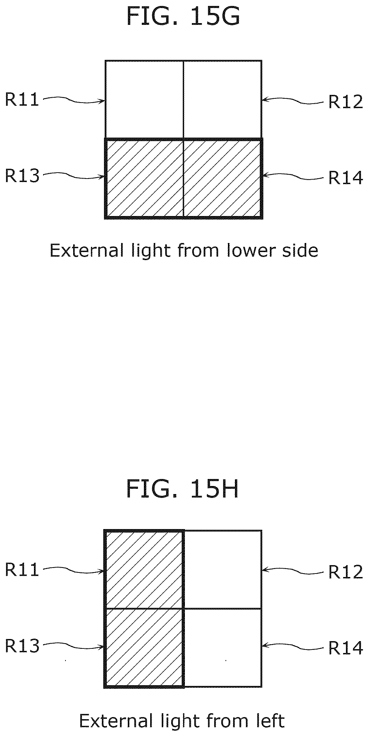

   FIG. 15G illustrates an example of the determination results of incident levels in the four divided areas when external light enters from the lower side.
[FIG. 15H]
   FIG. 15H illustrates an example of the determination results of incident levels in the four divided areas when external light enters from the left.
[FIG. 16A]

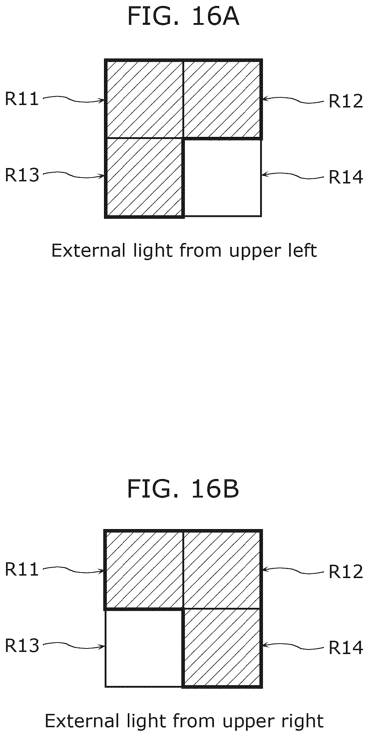

   FIG. 16A illustrates another example of the determination results of incident levels in the four divided areas when external light enters from the upper left.
[FIG. 16B]
   FIG. 16B illustrates another example of the determination results of incident levels in the four divided areas when external light enters from the upper right.
[FIG. 16C]

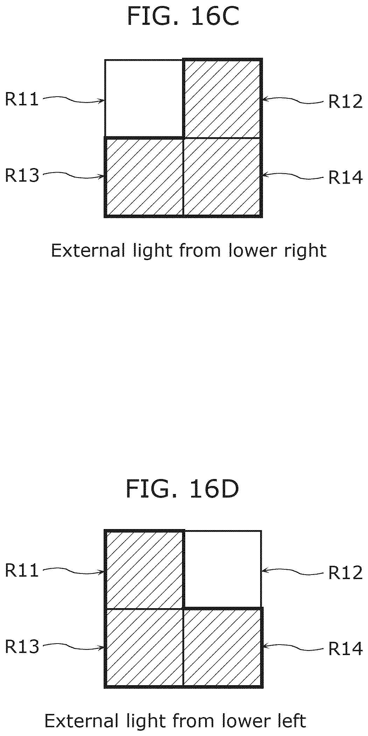

   FIG. 16C illustrates another example of the determination results of incident levels in the four divided areas when external light enters from the lower right.
[FIG. 16D]
   FIG. 16D illustrates another example of the determination results of incident levels in the four divided areas when external light enters from the lower left.
[FIG. 17A]

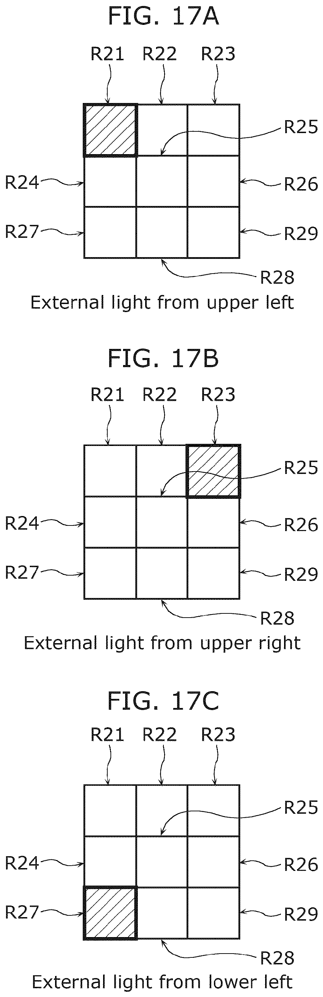

   FIG. 17A illustrates a first example of the determination results of incident levels in nine divided areas when external light enters from the upper left.
[FIG. 17B]
   FIG. 17B illustrates a first example of the determination results of incident levels in the nine divided areas when external light enters from the upper right.
[FIG. 17C]
   FIG. 17C illustrates a first example of the determination results of incident levels in the nine divided areas when external light enters from the lower left.
[FIG. 17D]

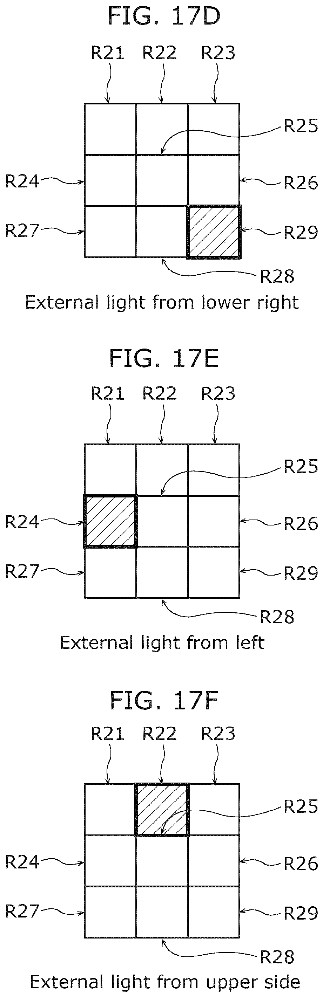

   FIG. 17D illustrates a first example of the determination results of incident levels in the nine divided areas when external light enters from the lower right.
[FIG. 17E]
   FIG. 17E illustrates a first example of the determination results of incident levels in the nine divided areas when external light enters from the left.
[FIG. 17F]
   FIG. 17F illustrates a first example of the determination results of incident levels in the nine divided areas when external light enters from the upper side.
[FIG. 17G]

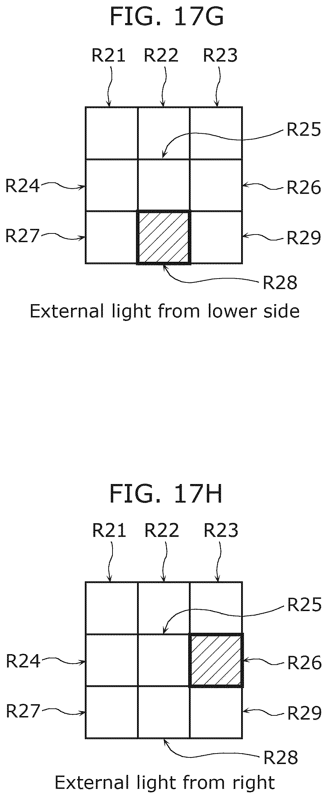

   FIG. 17G illustrates a first example of the determination results of incident levels in the nine divided areas when external light enters from the lower side.
[FIG. 17H]
   FIG. 17H illustrates a first example of the determination results of incident levels in the nine divided areas when external light enters from the right.
[FIG. 18A]

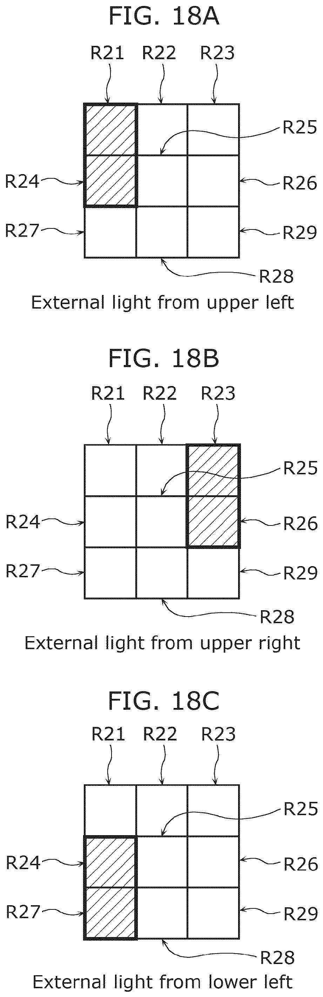

   FIG. 18A illustrates a second example of the determination results of incident levels in the nine divided areas when external light enters from the upper left.
[FIG. 18B]
   FIG. 18B illustrates a second example of the determination results of incident levels in the nine divided areas when external light enters from the upper right.
[FIG. 18C]
   FIG. 18C illustrates a second example of the determination results of incident levels in the nine divided areas when external light enters from the lower left.
[FIG. 18D]

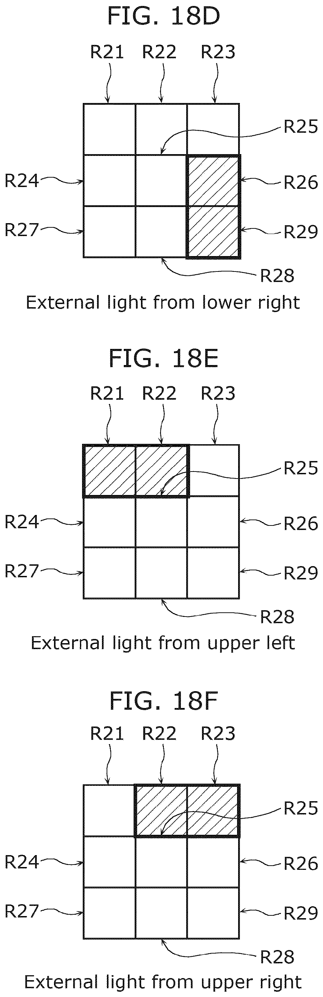

   FIG. 18D illustrates a second example of the determination results of incident levels in the nine divided areas when external light enters from the lower right.
[FIG. 18E]
   FIG. 18E illustrates a third example of the determination results of incident levels in the nine divided areas when external light enters from the upper left.
[FIG. 18F]
   FIG. 18F illustrates a third example of the determination results of incident levels in the nine divided areas when external light enters from the upper right.
[FIG. 18G]

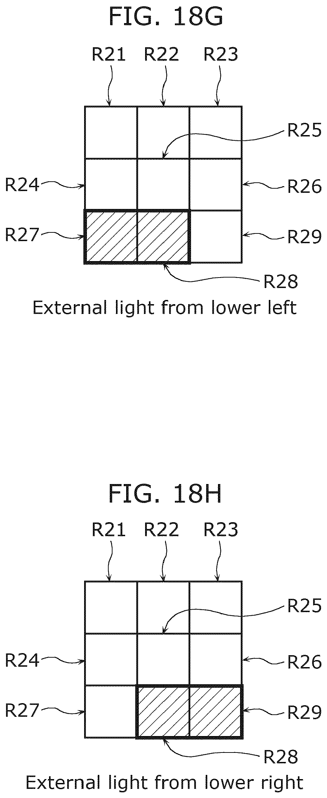

   FIG. 18G illustrates a third example of the determination results of incident levels in the nine divided areas when external light enters from the lower left.
[FIG. 18H]
   FIG. 18H illustrates a third example of the determination results of incident levels in the nine divided areas when external light enters from the lower right.
[FIG. 19A]

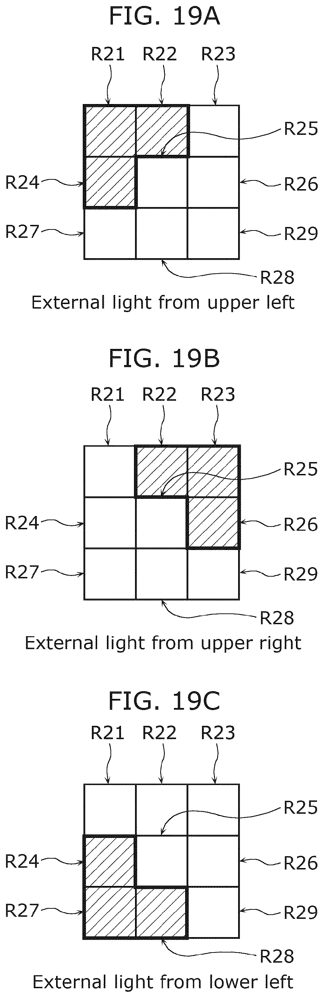

   FIG. 19A illustrates a fourth example of the determination results of incident levels in the nine divided areas when external light enters from the upper left.
[FIG. 19B]
   FIG. 19B illustrates a fourth example of the determination results of incident levels in the nine divided areas when external light enters from the upper right.
[FIG. 19C]
   FIG. 19C illustrates a fourth example of the determination results of incident levels in the nine divided areas when external light enters from the lower left.
[FIG. 19D]

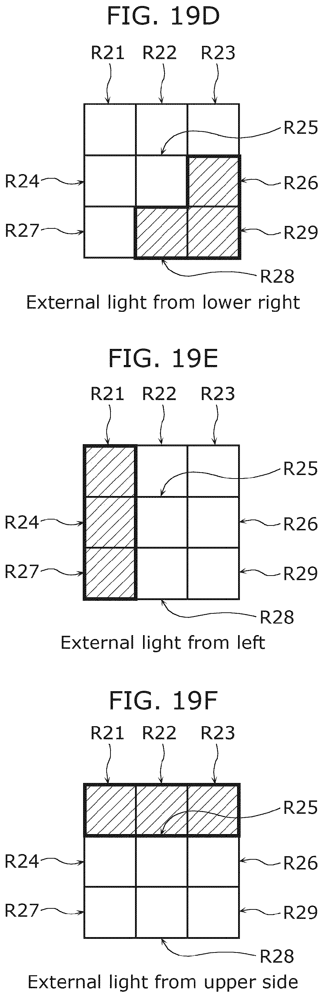

   FIG. 19D illustrates a fourth example of the determination results of incident levels in the nine divided areas when external light enters from the lower right.
[FIG. 19E]
   FIG. 19E illustrates a second example of the determination results of incident levels in the nine divided areas when external light enters from the left.
[FIG. 19F]
   FIG. 19F illustrates a second example of the determination results of incident levels in the nine divided areas when external light enters from the upper side.
[FIG. 19G]

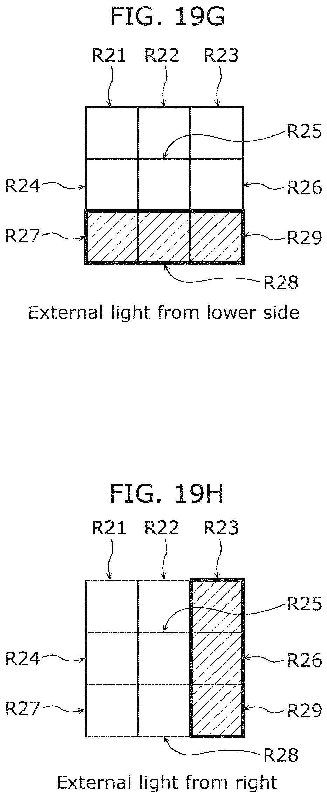

   FIG. 19G illustrates a second example of the determination results of incident levels in the nine divided areas when external light enters from the lower side.
[FIG. 19H]
   FIG. 19H illustrates a second example of the determination results of incident levels in the nine divided areas when external light enters from the right.
[FIG. 20A]

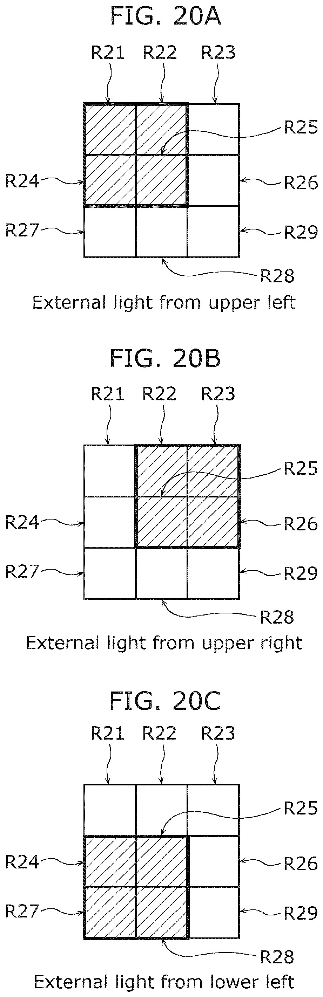

   FIG. 20A illustrates a fifth example of the determination results of incident levels in the nine divided areas when external light enters from the upper left.
[FIG. 20B]
   FIG. 20B illustrates a fifth example of the determination results of incident levels in the nine divided areas when external light enters from the upper right.
[FIG. 20C]
   FIG. 20C illustrates a fifth example of the determination results of incident levels in the nine divided areas when external light enters from the lower left.
[FIG. 20D]

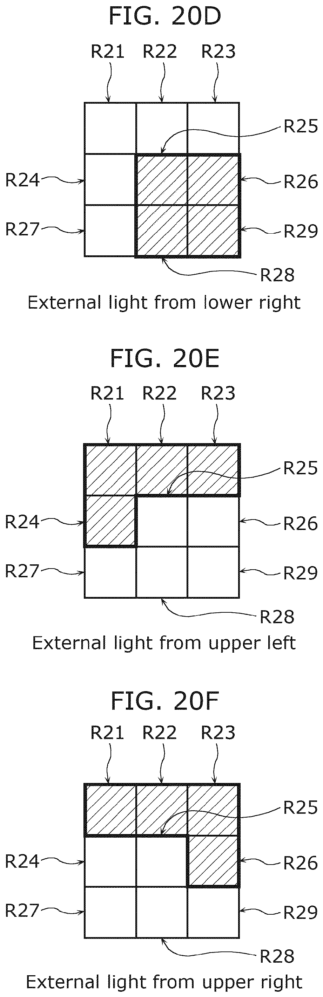

   FIG. 20D illustrates a fifth example of the determination results of incident levels in the nine divided areas when external light enters from the lower right.
[FIG. 20E]
   FIG. 20E illustrates a sixth example of the determination results of incident levels in the nine divided areas when external light enters from the upper left.
[FIG. 20F]
   FIG. 20F illustrates a sixth example of the determination results of incident levels in the nine divided areas when external light enters from the upper right.
[FIG. 20G]

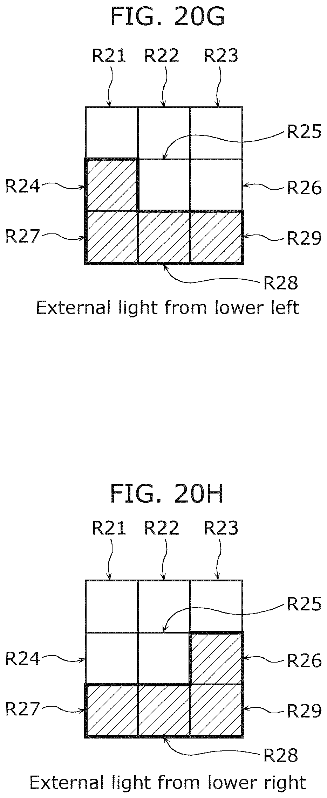

   FIG. 20G illustrates a sixth example of the determination results of incident levels in the nine divided areas when external light enters from the lower left.
[FIG. 20H]
   FIG. 20H illustrates a sixth example of the determination results of incident levels in the nine divided areas when external light enters from the lower right.
[FIG. 21A]

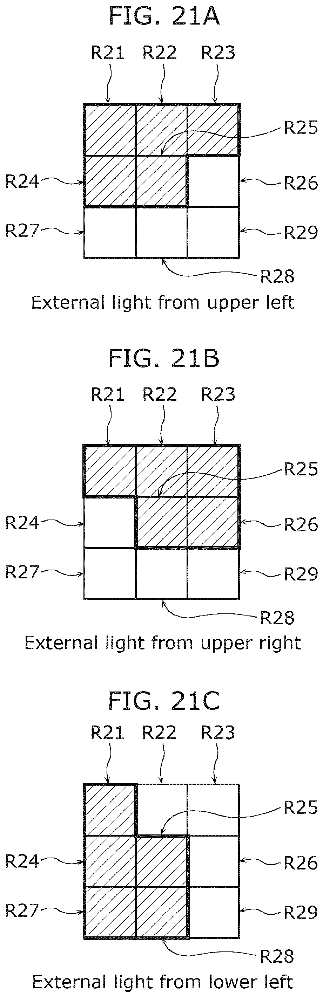

   FIG. 21A illustrates a seventh example of the determination results of incident levels in the nine divided areas when external light enters from the upper left.
[FIG. 21B]
   FIG. 21B illustrates a seventh example of the determination results of incident levels in the nine divided areas when external light enters from the upper right.
[FIG. 21C]
   FIG. 21C illustrates a seventh example of the determination results of incident levels in the nine divided areas when external light enters from the lower left.
[FIG. 21D]

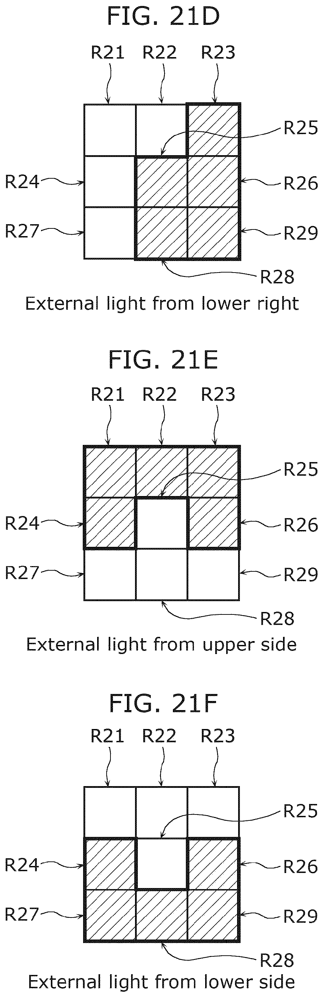

   FIG. 21D illustrates a seventh example of the determination results of incident levels in the nine divided areas when external light enters from the lower right.
[FIG. 21E]
   FIG. 21E illustrates a third example of the determination results of incident levels in the nine divided areas when external light enters from the upper side.
[FIG. 21F]
   FIG. 21F illustrates a third example of the determination results of incident levels in the nine divided areas when external light enters from the lower side.
[FIG. 21G]

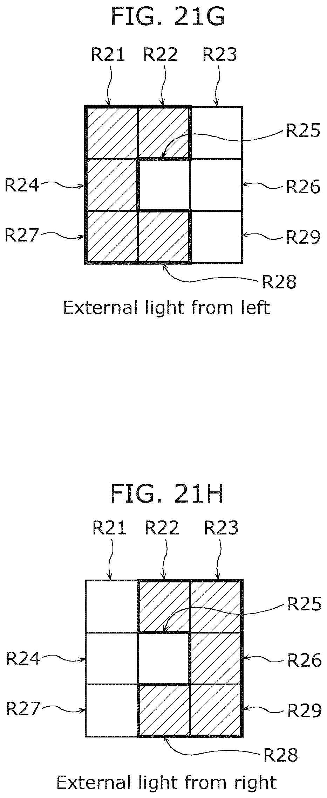

   FIG. 21G illustrates a third example of the determination results of incident levels in the nine divided areas when external light enters from the left.
[FIG. 21H]
   FIG. 21H illustrates a third example of the determination results of incident levels in the nine divided areas when external light enters from the right.
[FIG. 22]

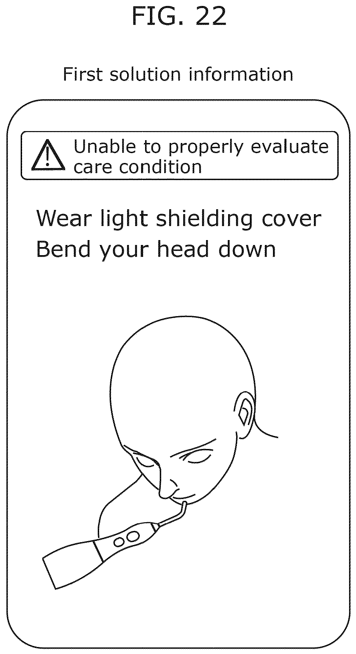

   FIG. 22 illustrates an example of first solution information according to Variation 2 of the embodiment.
[FIG. 23]

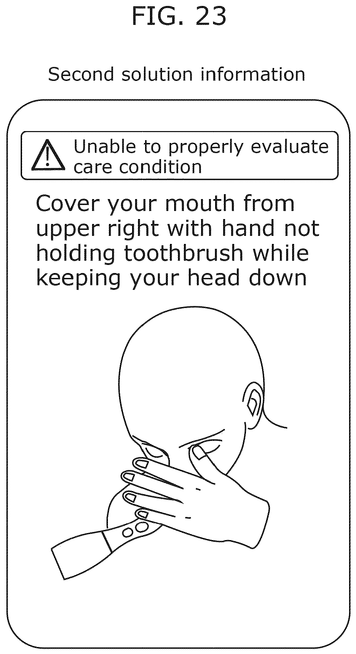

   FIG. 23 illustrates an example of second solution information according to Variation 2 of the embodiment.
[FIG. 24]

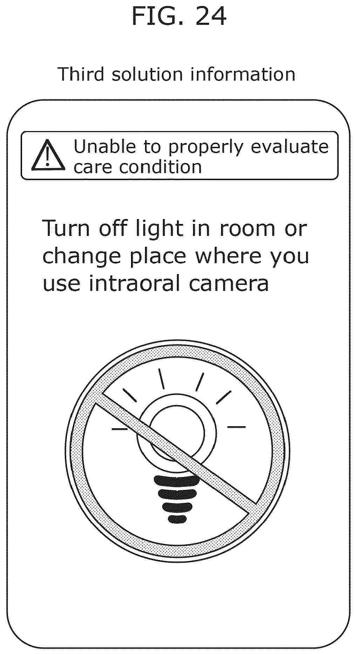

   FIG. 24 illustrates an example of third solution information according to Variation 2 of the embodiment.

### [Description of Embodiments]

### (Circumstances Leading to the Present Disclosure)

Before the explanations of the present disclosure, circumstances leading to the present disclosure are explained with reference to FIGS. 12 and 13. FIG. 12 illustrates a relationship between values based on respective pixel values and the number of pixels in a scene with the presence of external light. FIG. 13 illustrates a relationship between values based on respective pixel values and the number of pixels in a scene without external light. (a) in FIG. 12 and (a) in FIG. 13 each illustrates the relationship between the respective pixel values and the number of pixels of an image (RGB image) resulting from light received by an image sensor with or without the presence of external light. The vertical axis indicates the number of pixels, and the horizontal axis indicates the pixel value. (b) in FIG. 12 and (b) in FIG. 13 each illustrates the relationship between the number of pixels and the value of each parameter when HSV conversion has been performed on the image. The vertical axis indicates the number of pixels, and the horizontal axis indicates the values of the parameters. It should be noted that the parameters include h (hue), s (saturation), v (value), and y (luminance). Moreover, the horizontal axis is indicated by values for 8 bits.

It should be noted that the results illustrated in FIGS. 12 and 13 are results for the case where an intraoral camera for capturing an image of an oral cavity includes a blue-light blocking filter, which is described later. Moreover, the presence of external light means that external light is present inside an oral cavity, and other expressions such as "external light is entering" are also stated below. The absence of external light means that external light is not present inside the oral cavity, and other expressions such as "external light is not entering" are also stated below. It should be noted that the meaning of the absence of external light may also include the situation in which external light is substantially not present, in addition to the situation in which there is no external light.

As illustrated in (a) in FIG. 12, when external light is present, there are an outstandingly large number of pixels with high pixel values in red (see the dashed line frame). For instance, there are a large number of pixels in which pixel values in only red among red, blue, and green are high (in which Rch is high). In this case, an entire image becomes reddish.

As illustrated in (b) in FIG. 12, when external light is present, there are a large number of pixels in which the value of only Value among Hue, Saturation, Value, and Luminance is high (in which V is high) (see the dashed line frame). That is, the Value of the image is high.

Here, as already known in the quantitative light-induced fluorescence (QLF), it is known that bacteria contained in dental plaque fluoresces in pink with red tinge (emits excited fluorescence) in response to exposure to blue light. Thus, if external light is present and the entire image becomes reddish, it will be difficult to detect a dental plaque area from the image. For instance, in a situation where an area without dental plaque is being exposed to external light, there is a possibility of detecting that the area without dental plaque contains dental plaque due to the effects of the external light.

As illustrated in (a) in FIG. 13, when external light is not present, there are a small number of pixels having high red pixel values (there are a small number of pixels with high Rch) (see the dashed line frame).

As illustrated in (b) in FIG. 13, when external light is not present, there are a small number of pixels with high Value (there are a small number of pixels with high V) (see the dashed line frame).

As described above, depending on whether external light is present, there is a large difference in the number of pixels having high pixel values of red. The inventors of the present application focused on the number of pixels having high pixel values of red and found that the number of the pixels can be used as an example of a method of determining whether external light is present. Then, the inventors of the present application diligently studied, for example, an image processing method capable of notifying, using the number of pixels having high pixel values of red, that a dental plaque area may have been detected by error due to the external light that has entered the oral cavity.

An image processing method according to the first aspect of the present disclosure is an image processing method for displaying a dental plaque area based on an RGB image obtained by capturing an image of a tooth and dental plaque fluorescing in response to exposure to light including a blue-light wavelength range, the image processing method including: obtaining the incident level of external light entering an imaging area of a first RGB image; performing dental plaque detection processing based on the first RGB image; and changing the display mode of a dental plaque detection result according to the incident level of the external light which has entered the imaging area of the first RGB image.

In this way, it is possible to change the display mode of the dental plaque detection result according to whether external light is entering the oral cavity. When external light is entering the oral cavity, a dental plaque area included in the dental plaque detection result may have been detected by error. When for instance external light is entering the oral cavity, by changing to the display mode that can notify that external light is entering, it is possible to achieve an image processing method capable of notifying that the dental plaque area may have been detected by error due to the external light that entered the oral cavity.

Moreover, for instance, an image processing method according to the second aspect is the image processing method according to the first aspect, in which when the incident level is not lower than or equal to a predetermined value, an image in which information indicating that the external light is entering is superposed onto an image displaying the dental plaque detection result may be displayed.

In this way, it is possible to notify a user that there is a possibility of external light entering the oral cavity, that is, the dental plaque area may have been detected by error.

Moreover, for instance, an image processing method according to the third aspect is the image processing method according to the first or second aspect, in which when the incident level is lower than or equal to the predetermined value, an image in which information indicating that the external light is not entering is superposed onto an image displaying the dental plaque detection result may be displayed.

In this way, it is possible to notify the user that external light is not entering, that is, there is a high possibility that the dental plaque area has been properly detected.

Moreover, for instance, an image processing method according to the fourth aspect is the image processing method according to the first aspect, in which when the incident level is not lower than or equal to a predetermined value, displaying of the dental plaque detection result may be prohibited.

In this way, if there is a possibility of external light entering, it is possible to suppress the dental plaque detection result that may include an incorrect detection result from being presented to the user.

Moreover, for instance, an image processing method according to the fifth aspect is the image processing method according to one of the first to fourth aspects, in which the incident level may be obtained based on a detection result of a detector that detects the external light entering the imaging area of the first RGB image.

In this way, since whether external light is present is directly detected by the detector, it is possible to more reliably detect whether external light is present. Moreover, it is possible to reduce the amount of processing required when a processing device that performs the image processing method determines whether external light is present.

Moreover, for instance, an image processing method according to the sixth aspect is the image processing method according to one of the first to fifth aspects, in which the incident level may be calculated based on a first red pixel count indicating the total number of red pixels having red pixel values greater than or equal to a first pixel value in the first RGB image.

In this way, it is possible to determine whether external light is present in accordance with the first red pixel count in which a difference in the pixel count tends to occur according to whether external light is present.

Moreover, for instance, an image processing method according to the seventh aspect is the image processing method according to the sixth aspect, in which the incident level may include a first incident level, the first red pixel count may include, for each of pixel values greater than or equal to the first pixel value, a first pixel count indicating the total number of red pixels having the pixel value, and a green pixel count indicating the total number of green pixels having green pixel values greater than or equal to the first pixel value may include, for each of the pixel values greater than or equal to the first pixel value, a second pixel count indicating the total number of green pixels having the pixel value. The image processing method may include: subtracting the second pixel count from the first pixel count in each of the pixel values greater than or equal to the first pixel value; calculating the first incident level in accordance with a first integrated value calculated by integrating one or more positive first subtraction values among first subtraction values each of which is calculated by subtracting the second pixel count from the first pixel count; and changing the display mode of the dental plaque detection result according to whether the first incident level is lower than or equal to a first predetermined value.

In this way, whether external light is present is determined using the first incident level based on the first integrated value, and the display mode of the dental plaque detection result can be changed according to the result of determination as to whether external light is present.

Moreover, for instance, an image processing method according to the eighth aspect is the image processing method according to the sixth aspect, in which the incident level may include a first incident level, the first red pixel count may include a first pixel count indicating the total number of red pixels having pixel values greater than or equal to the first pixel value, and a green pixel count indicating the total number of green pixels having green pixel values less than or equal to a fourth pixel value lower than the first pixel value may include a second pixel count indicating the total number of green pixels having pixel values less than or equal to the fourth pixel value. The image processing method may include: subtracting the second pixel count from the first pixel count; when a first subtraction value calculated by subtracting the second pixel count from the first pixel count is a positive value, calculating the first incident level in accordance with the first subtraction value; and changing the display mode of the dental plaque detection result according to whether the first incident level is lower than or equal to a first predetermined value.

In this way, whether external light is present is determined using the first incident level based on the first integrated value, and the display mode of the dental plaque detection result can be changed according to the result of determination as to whether external light is present.

Moreover, for instance, an image processing method according to the ninth aspect is the image processing method according to the seventh aspect, in which the incident level may include a second incident level, and a second red pixel count indicating the total number of red pixels having red pixel values greater than or equal to a second pixel value higher than the first pixel value may include, for each of pixel values greater than or equal to the second pixel value, a third pixel count indicating the total number of red pixels having the pixel value. The image processing method may include: subtracting the second pixel count from the third pixel count in each of the pixel values greater than or equal to the second pixel value; calculating the second incident level in accordance with a second integrated value calculated by integrating one or more positive second subtraction values among second subtraction values each of which is calculated by subtracting the second pixel count from the third pixel count; and changing the display mode of the dental plaque detection result according to whether the second incident level is lower than or equal to a second predetermined value.

In this way, by using the second integrated value, even when external light is entering a local area (for example, a local area of a tooth is glossy), it is possible to determine that external light is present. Accordingly, it is possible to suppress error detection in which although external light is entering the local area, it is determined that external light is not present.

Moreover, for instance, an image processing method according to the tenth aspect is the image processing method according to one of the seventh to ninth aspects, in which the incident level may include a third incident level. The image processing method may include: generating a V image having V values obtained by performing HSV conversion on the pixel values of the first RGB image; calculating the third incident level in accordance with an integrated value indicating the total number of pixels whose V values are greater than or equal to a third pixel value; and changing the display mode of the dental plaque detection result further according to whether the third incident level is lower than or equal to a third predetermined value.

In this way, it is possible to change the display mode of the dental plaque detection result in accordance with the number of red pixels and Value.

Moreover, for instance, an image processing method according to the eleventh aspect is the image processing method according to the tenth aspect, in which when the first incident level is at most the first predetermined value and at least a fourth predetermined value lower than the first predetermined value and when the third incident level is at most the third predetermined value and at least a fifth predetermined value lower than the third predetermined value, an image in which information suggesting blocking light is superposed onto an image displaying the dental plaque detection result may be displayed.

In this way, if it is uncertain whether external light is entering, blocking light is suggested. By blocking light when external light is entering, it is possible to suppress the effects of the external light. Moreover, by blocking light when external light is not entering, it is possible to confirm that external light was not entering.

Moreover, for instance, an image processing method according to the twelfth aspect is the image processing method according to the sixth aspect, which may include generating a V image having V values obtained by performing HSV conversion on the pixel values of the first RGB image; and calculating the incident level in accordance with an integrated value indicating the total number of pixels whose V values are greater than or equal to the first pixel value.

In this way, whether external light is present can be determined in accordance with the V values.

Moreover, for instance, an image processing method according to the thirteenth aspect is the image processing method according to one of the second to fourth aspects, in which the predetermined value may be set according to the lighting environment of a space where the first RGB image is to be captured.

In this way, since the predetermined value suitable for each lighting environment is set, even if the lighting environment is changed, it is possible to accurately determine whether external light is present.

Moreover, for instance, an image processing method according to the fourteenth aspect is the image processing method according to one of the second to fourth aspects, which may include dividing the first RGB image into a plurality of areas; obtaining the incident level of the external light entering each of the plurality of areas into which the first RGB image has been divided; and changing the display mode of the dental plaque detection result according to the result of determination as to whether the incident level in each of the plurality of areas is lower than or equal to the predetermined value.

In this way, since whether external light is present is determined in a narrower area, even if external light is entering a local area, it is possible to accurately determine that external light is present. Accordingly, it is possible to suppress error detection in which although external light is entering the local area, it is determined that external light is not present.

Moreover, for instance, an image processing method according to the fifteenth aspect is the image processing method according to one of the second to fourth aspects may include displaying solution information that depends on the incident level to suppress the effect of the external light.

In this way, by the user performing a solution indicated in the solution information, it is possible to suppress the incident level of external light. Accordingly, it is possible to suppress external light from entering the oral cavity.

Moreover, for instance, an image processing method according to the sixteenth aspect is the image processing method according to the fifteenth aspect which may include: dividing the first RGB image into a plurality of areas; obtaining the incident level of the external light entering each of the plurality of areas into which the first RGB image has been divided; and displaying solution information that depends on at least one of the total number of one or more areas, among the plurality of areas, in each of which the incident level exceeds the predetermined value or the positions of the one or more areas among the plurality of areas.

In this way, it is possible to display the solution information that depends on the number of areas the external light is entering and the incident direction of the external light. Accordingly, it is possible to effectively suppress the external light from entering the oral cavity.

Moreover, for instance, an image processing method according to the seventeenth aspect is the image processing method according to the sixteenth aspect which may include: a determiner that determines whether a user has responded to the solution information; after the user is determined to have responded to the solution information, obtaining post-response incident levels in the plurality of areas in a second RGB image obtained by capturing an image of the tooth and the dental plaque fluorescing in response to exposure to the light including the blue-light wavelength range; and displaying different solution information based on the post-response incident levels in the plurality of areas, the different solution information being information different from the solution information.

In this way, it is possible to display another solution information based on external light reduction effects obtained by responding to the displayed solution information. For instance, if the external light reduction effects obtained by responding to the displayed solution information are not sufficient, it is possible to display another solution information that can further suppress the effects of the external light. Accordingly, it is possible to more reliably suppress the external light from entering the oral cavity.

Moreover, for instance, an image processing method according to the eighteenth aspect is the image processing method according to the seventeenth aspect, in which the different solution information may be information that depends on at least one of the total number of one or more areas, among the plurality of areas, in each of which a post-response incident level exceeds the predetermined value or the positions of the one or more areas among the plurality of areas.

In this way, it is possible to display another solution information that depends on the number of areas the external light is entering and the incident direction of the external light. Accordingly, it is possible to more effectively suppress the external light from entering the oral cavity.

Moreover, for instance, an image processing method according to the nineteenth aspect is the image processing method according to one of the sixteenth to eighteenth aspects which may include: determining the incident direction of the external light in accordance with the positions of the one or more areas in each of which the incident level exceeds the predetermined value among the plurality of areas, in which the solution information may include information on the incident direction of the external light.

In this way, it is possible to display the solution information that especially focuses on the incident direction of the external light.

An image processing system according to the twentieth aspect of the present disclosure is an image processing system for displaying a dental plaque area based on an RGB image obtained by capturing an image of a tooth and dental plaque fluorescing in response to exposure to light including a blue-light wavelength range, the image processing system including: an obtainer that obtains the incident level of external light entering an imaging area of the RGB image; a dental plaque detector that performs dental plaque detection processing based on the RGB image; and a display controller that changes the display mode of a dental plaque detection result according to the incident level of the external light which has entered the imaging area of the RGB image. Moreover, a program according to the twenty-first aspect of the present disclosure is a program for causing a computer to execute the image processing method according to one of the first to nineteenth aspects.

In this way, effects similar to those provided by the above image processing method are provided.

It should be noted that these general or specific aspects may be embodied as a system, a method, an integrated circuit, a computer program, or a computer-readable recording medium such as a CD-ROM or may be embodied as any combination of the system, the method, the integrated circuit, the computer program, and the recording medium.

It should be noted that the embodiments described below each indicate a comprehensive or specific example. The numerical values, shapes, constituent elements, arrangement and connection of the constituent elements, steps, order of steps, and other details indicated in the embodiments described below are merely examples, and do not intend to limit the present disclosure. Moreover, the constituent elements not recited in the independent claims, among those described in the embodiments below are described as optional constituent elements.

Moreover, the figures are schematic illustrations and are not necessarily precise depictions. Accordingly, for instance, the scales used in the figures need not necessarily be the same. Moreover, in the figures, substantially the same elements are assigned the same reference signs, and overlapping explanations are omitted or simplified.

Moreover, in the specification, terms indicating relationships between elements such as identical, terms indicating the shapes of elements such as L-shaped, U-shaped, and rectangular, numerical values, and a numerical value range are not expressions indicating only strict meanings but expressions intended to include practically equivalent ranges such as a difference of around several percentages (or around 10%).

Moreover, in the specification, unless otherwise noted, ordinal numerals such as the first and the second do not indicate the number or order of constituent elements, but are used to avoid the mix-up of constituent elements of the same kind and distinguish one from another.

### [Embodiment]

Hereinafter, an intraoral camera system and an image processing method according to the embodiment are described with reference to FIGS. 1 to 10.

### [1. Configuration of Intraoral Camera System]

First, a configuration of an intraoral camera system including an intraoral camera according to the embodiment is described with reference to FIGS. 1 to 3. FIG. 1 is a perspective view of intraoral camera 10 of the intraoral camera system according to the embodiment.

As illustrated in FIG. 1, intraoral camera 10 includes a toothbrush-shaped case that can be handled by one hand. The case includes head 10a, handle 10b, and neck 10c. Head 10a is put inside the oral cavity of a user when a dentition image is captured. Handle 10b is designed to be held by the user. Neck 10c connects head 10a and handle 10b.

Imaging unit 21 is incorporated into head 10a and neck 10c. Imaging unit 21 includes an image sensor (not illustrated) and a lens (not illustrated) that are disposed on optical axis LA thereof.

The image sensor is an imaging device, such as a complementary metal-oxide-semiconductor (CMOS) sensor or a charge coupled device (CCD), and the lens forms an image of a tooth. The image sensor outputs a signal (image data) corresponding to the formed image to an external destination. A dentition image captured by the image sensor is an example of an RGB image. Moreover, the dentition image may be an image of the side surface of a tooth and an image of the occlusal surface of a tooth. Moreover, the side surface of the tooth may be an internal side (lingual) surface and an external side (buccal) surface.

Imaging unit 21 may include a light blocking filter that blocks light with a color emitted from an illuminator and passes fluorescence that dental plaque emits in response to exposure to the light. In the embodiment, imaging unit 21 may include a blue-light blocking filter that blocks the light components of a blue wavelength contained in light that will enter image sensor 14. When teeth are irradiated with light including a blue-light wavelength range to detect dental plaque, when the light including the blue-light wavelength range is intensified to intensify the excitation fluorescence of the dental plaque, an entire RGB image takes on a blue tinge. In the state, a blue pixel value is dominant over a red pixel value and a green pixel value. Thus, the accuracy of determination may decrease when determining whether external light is present by using the red pixel value and the green pixel value. To deal with the issue, a blue-light blocking filter blocks a light ray including the blue-light wavelength range among light rays yet to enter the image sensor. It should be noted that imaging unit 21 need not include a blue-light blocking filter.

Moreover, intraoral camera 10 includes first to fourth light emitting diodes (LEDs) 23A to 23D as illuminators (illumination devices) that irradiate a target tooth during image capturing. First to fourth LEDs 23A to 23D emit light with a color (for example, light with a single color), exposure to which causes emission of fluorescence by dental plaque. First to fourth LEDs 23A to 23D are, for example, blue LEDs that emit blue light having a wavelength peak of 405 nm. It should be noted that first to fourth LEDs 23A to 23D may be light sources that emit light including the blue-light wavelength range and are not limited to blue LEDs.

FIG. 2 illustrates a schematic configuration of the intraoral camera system according to the embodiment.

As illustrated in FIG. 2, the intraoral camera system according to the embodiment is schematically configured to cause imaging unit 21 to capture an image of fluorescence that dental plaque emits in response to exposure to light from illuminator 23.

As illustrated in FIG. 2, the intraoral camera system includes intraoral camera 10 and portable terminal 50. The intraoral camera system is an example of an image processing system.

The intraoral camera system is configured, when a dental plaque area is detected on the basis of an image (RGB image) captured by intraoral camera 10 in a state in which external light was entering an oral cavity, to be able to notify that the dental plaque area may have been detected by error due to the external light that entered the oral cavity. The notification may be to display, on an image, information indicating that the dental plaque area may have been detected by error (see FIG. 9 described later, for example), and may be to output, by sound, light, or other methods, the information indicating the dental plaque area may have been detected by error. Moreover, external light is light other than light emitted by illuminator 23, and examples of external light include illumination light and sunlight. The external light may include, for example, light with the same color as fluorescence emitted by dental plaque.

Intraoral camera 10 includes hardware 20, signal processor 30, communicator 40.

Hardware 20 is a physical element included in intraoral camera 10, and includes imaging unit 21, sensor 22, illuminator 23, and operation interface 24.

Imaging unit 21 generates image data by capturing an image of a tooth inside the oral cavity of the user. Imaging unit 21 receives a control signal from camera controller 31, performs, in accordance with the received control signal, an operation such as image capturing, and outputs, to image processor 32, image data such as a video or a still image obtained by the image capturing. Imaging unit 21 includes the above image sensor, light blocking filter, and lens. Imaging unit 21 generates the image data according to the light that has transmitted through the light blocking filter. Moreover, the image data is a dentition image showing teeth, and may be a dentition image showing at least one tooth.

Sensor 22 detects external light entering the imaging area of an RGB image. For instance, sensor 22 detects whether external light is entering the oral cavity. For instance, sensor 22 is disposed near imaging unit 21. As with imaging unit 21, sensor 22 may be disposed in head 10a of intraoral camera 10, for example. That is, when imaging unit 21 captures an image, sensor 22 is put inside the oral cavity of the user. Sensor 22 is an example of a detector.

Illuminator 23 irradiates, with light (blue light in the embodiment), the area to be/being captured by imaging unit 21 among a plurality of areas inside the oral cavity. Illuminator 23 includes first to fourth LEDs 23A to 23D described above. For instance, first to fourth LEDs 23A to 23D irradiate the imaging area with light rays in mutually different directions. In this way, it is possible to suppress a shadow from occurring on the imaging area.

Each of first to fourth LEDs 23A to 23D is configured such that at least light control is possible. Each of first to fourth LEDs 23A to 23D may be configured such that light control and color control are possible. First to fourth LEDs 23A to 23D are disposed to surround imaging unit 21.

The irradiation intensity (light emission intensity) of illuminator 23 is controlled according to the imaging area. The irradiation intensities of first to fourth LEDs 23A to 23D may be controlled uniformly and may be controlled to be different from one another. It should be noted that the number of the LEDs of illuminator 23 is not limited to a particular number and may be one or at least five. Moreover, illuminator 23 is not limited to including LEDs as light sources and may include another light source.

Operation interface 24 receives user input. Operation interface 24 is configured as, for example, a press button and may be an element that receives an instruction by sound.

Moreover, hardware 20 may further include a battery (e.g., a secondary battery) for supplying power to the constituent elements of intraoral camera 10, a coil for use in wireless charging by an external charger connected to a commercial power supply, and an actuator necessary to perform at least one of composition adjustment or focus adjustment.

Signal processor 30 includes functional elements achieved by using, for example, a central processing unit (CPU) or a microprocessor unit (MPU) which performs various processing tasks described later and memory 35 such as read only memory (ROM) or random access memory (RAM) storing programs for causing the functional elements to perform the various processing tasks. Signal processor 30 includes camera controller 31, image processor 32, controller 33, illumination controller 34, and memory 35.

Camera controller 31 is included in, for example, handle 10b of intraoral camera 10, and controls imaging unit 21. For instance, camera controller 31 controls at least one of the aperture or shutter speed of imaging unit 21 according to a control signal from image processor 32.

Image processor 32 is included in, for example, handle 10b of intraoral camera 10. Image processor 32 obtains a dentition image (image data) captured by imaging unit 21, performs image processing on the obtained dentition image, and outputs the dentition image which has undergone the image processing to camera controller 31 and controller 33. Moreover, image processor 32 may output the dentition image which has undergone the image processing to memory 35, thereby storing, in memory 35, the dentition image which has undergone the image processing.

Image processor 32 is configured as, for example, a circuit, and performs, on the dentition image, the image processing such as noise removal, automatic white balance (AWB) processing, and outline enhancement processing. The AWB processing may be performed mainly on, for example, a tooth area in the dentition image.

It should be noted that the dentition image (the dentition image after the image processing) output from image processor 32 may be transmitted to portable terminal 50 via communicator 40, and the transmitted dentition image may be displayed on touch screen 54 of portable terminal 50. In this way, it is possible to present the dentition image to the user.

Controller 33 detects a dental plaque area on the basis of the image data on which image processor 32 has performed the image processing. Controller 33 may identify a dental plaque area shown in the image data, using a machine learning model trained to output a dental plaque area shown in image data in response to inputting of the image data.

Illumination controller 34 is included in, for example, handle 10b of intraoral camera 10 and controls on and off of first to fourth LEDs 23A to 23D. Illumination controller 34 is configured as, for example, a circuit. When for instance the user performs an operation to activate intraoral camera 10 on touch screen 54 of portable terminal 50, a corresponding signal is transmitted from portable terminal 50 to signal processor 30 via communicator 40. Illumination controller 34 of signal processor 30 turns on first to fourth LEDs 23A to 23D in accordance with the received signal.

Aside from the above programs, for example, the dentition image (image data) captured by imaging unit 21 is stored in memory 35. As a non-limiting example, memory 35 is embodied as semiconductor memory such as ROM and RAM.

Communicator 40 is a wireless communication module that performs wireless communication with portable terminal 50. Communicator 40 is included in, for example, handle 10b of intraoral camera 10 and performs wireless communication with portable terminal 50 in accordance with the control signal from signal processor 30. Communicator 40 performs, with portable terminal 50, wireless communication complying with an existing communication standard, such as Wi-Fi (registered trademark) or Bluetooth (registered trademark). A dentition image showing teeth is transmitted from intraoral camera 10 to portable terminal 50 via communicator 40, and an operation signal is transmitted from portable terminal 50 to intraoral camera 10 via communicator 40.

For instance, portable terminal 50 displays a dental plaque area on the basis of an RGB image showing teeth and dental plaque fluorescing in response to exposure to light including a blue-light wavelength range. Moreover, portable terminal 50 functions as the user interface of the intraoral camera system.

FIG. 3 is a block diagram illustrating a functional configuration of portable terminal 50 according to the embodiment.

As illustrated in FIG. 3, portable terminal 50 includes obtainer 51, controller 52, display controller 53, and touch screen 54. Portable terminal 50 includes, for example, a processor and memory. The memory is, for example, ROM and RAM, and can store programs to be executed by a processor. Obtainer 51, controller 52, and display controller 53 can be achieved by using, for example, a processor that executes the programs stored in the memory. Portable terminal 50 may be achieved as, for example, a smartphone or a tablet terminal capable of performing wireless communication.

Obtainer 51 obtains an RGB image from intraoral camera 10. Obtainer 51 is, for example, a wireless communication module that performs wireless communication.

Controller 52 performs the processing for detecting a dental plaque area on the basis of an RGB image. Controller 52 performs level determination processing for determining the incident level of external light entering the imaging area of the image and the dental plaque detection processing for detecting a dental plaque area on the basis of the RGB image. Controller 52 is an example of a dental plaque detector.

Here, the level determination processing of controller 52 is described with reference to FIGS. 4 to 6.

Controller 52 determines whether external light is entering on the basis of at least one of the number of pixels of a predetermined color in the RGB image or the number of pixels having Value greater than or equal to a predetermined value. In the embodiment, as a first method, by using the tendency that the entire image becomes reddish when external light is entering, controller 52 may determine whether external light is entering on the basis of a first red pixel count including, for each of pixel values greater than or equal to a first pixel value, a first pixel count indicating the number of red pixels having the pixel value. Moreover, in the embodiment, controller 52 may determine whether external light is entering further on the basis of a first green pixel count including, for each of the pixel values greater than or equal to the first pixel value, a second pixel count indicating the number of green pixels having the pixel value.

In each of the pixel values greater than or equal to the first pixel value, controller 52 subtracts the second pixel count having the pixel value from the first pixel count having the pixel value, integrates one or more positive first subtraction values among first subtraction values each of which is calculated by subtracting the second pixel count from the first pixel count, and calculates a first incident level on the basis of a first integrated value obtained by integrating the one or more positive first subtraction values. For instance, controller 52 may calculate, as the first incident level, a value obtained by dividing the first integrated value by the total number of pixels. It should be noted that to calculate the incident level is an example of obtainment of the incident level.

Moreover, as a second method, controller 52 may determine whether external light is entering by evaluating the number of pixels in each of which a green pixel value is smaller than a red pixel value. In this case, controller 52 may determine whether external light is entering in accordance with the first red pixel count including a first pixel count indicating the number of red pixels having pixel values greater than or equal to the first pixel value and the green pixel count including a second pixel count indicating the number of green pixels having pixel values (green pixel values) less than or equal to a fourth pixel value lower than the first pixel value. Moreover, controller 52 may subtract the second pixel count from the first pixel count, and when a first subtraction value calculated by subtracting the second pixel count from the first pixel count is a positive value, controller 52 may calculate a first incident level in accordance with the first subtraction value. For instance, controller 52 may calculate, as the first incident level, a value calculated by dividing the first subtraction value by the total number of pixels. It should be noted that the first red pixel count is not limited to the pixel count described above and may be the number of pixels in which a red pixel value is relatively large in comparison with a green pixel value. The green pixel count may be the number of pixels in which a green pixel value is relatively large in comparison with a red pixel value.

Then, controller 52 changes the display mode of the dental plaque detection result according to the incident level of external light. Specifically, controller 52 determines whether the first incident level is lower than or equal to a first predetermined value, and outputs a determination result to display controller 53. In this way, controller 52 changes the display mode of the dental plaque detection result according to the determination result. It should be noted that the first pixel value and the first predetermined value are appropriately set according to the lighting environment of the space in which to use intraoral camera 10. For instance, the first pixel value is set according to the lighting environment in which an RGB image is to be captured. For instance, when illuminance is 800 lux and the pixel value is expressed as 8 bits, the first pixel value may be 130. Moreover, the first pixel value may be set to a numerical value higher than 130 in order to suppress the determination that external light is present despite the absence of external light, that is, the stop of dental plaque determination despite the absence of external light.

Moreover, controller 52 generates a V image having V values (Value) obtained by performing HSV conversion on the pixel values of the RGB image, and calculates a third incident level in accordance with a third integrated value indicating the number of pixels whose V values are greater than or equal to a third pixel value. Controller 52 may obtain, as the third incident level, a value calculated by dividing the third integrated value by the total number of pixels. Then, controller 52 determines whether the third incident level is lower than or equal to a third predetermined value, and outputs a determination result to display controller 53. In this way, controller 52 changes the display mode of the dental plaque detection result according to the determination result. It should be noted that the third pixel value and the third predetermined value are appropriately set according to the lighting environment of the space in which to use intraoral camera 10. For instance, when illuminance is 800 lux and the pixel value is expressed as 8 bits, the third pixel value may be 250.

FIG. 4 is a first figure with the plotted results of determination as to whether external light is present according to the embodiment. The horizontal axis in FIG. 4 indicates the first incident level (indicating "R-G Range 1" in FIG. 4), and the vertical axis indicates the third incident level ("V range" in FIG. 4). Moreover, in FIG. 4, the circles with the hatching pattern indicate the first incident level and the third incident level obtained from an RGB image captured when external light was present. Meanwhile, the circles without the hatching pattern indicate the first incident level and the third incident level obtained from an RGB image captured without external light.

Area A in FIG. 4 is the area in which almost all of the plotted circles are circles indicating the absence of external light. As such, by determining that external light is not present when the first incident level is lower than or equal to threshold r2 (for example, approximately 0.18), it is possible to determine with high accuracy that external light is not present. Likewise, by determining that external light is not present when the third incident level is lower than or equal to threshold v2 (for example, approximately 0.24), it is possible to determine with high accuracy that external light is not present. In this case, since external light is not present, the dental plaque detection processing is performed.

Area C in FIG. 4 is the area in which all of the plotted circles are circles indicating the presence of external light. As such, by determining that external light is present when the first incident level is greater than or equal to threshold r1 (for example, 0.45), it is possible to determine with high accuracy that external light is present. Likewise, by determining that external light is present when the third incident level is greater than or equal to threshold v1 (for example, 0.45), it is possible to determine with high accuracy that external light is present. In this case, since there is a high possibility of detecting a dental plaque area by error due to the external light, the dental plaque detection processing will not be performed. Threshold r1 is an example of the first predetermined value, and threshold r2 is an example of a fourth predetermined value. Moreover, threshold v1 is an example of the third predetermined value, and threshold v2 is an example of a fifth predetermined value.

Area B in FIG. 4 is the area where both circles indicating the presence of external light and circles indicating the absence of external light are plotted. In this case, although the dental plaque detection processing is performed, there is a possibility of detecting a dental plaque area by error due to the external light. Thus, the display mode of the dental plaque detection result is changed depending on whether the area in which at least one of the first incident level or the third incident level is plotted is area B or area A. Specifically, when the area is area B, the dental plaque detection result is displayed in the display mode that notifies a user that a dental plaque area may have been detected by error.

It should be noted that from the results shown in FIG. 4, when for instance the following determination expression is satisfied, controller 52 may determine that external light is present.

In using the first method, for instance, when a value (the number of pixels) calculated by subtracting the number of green pixels having a pixel value of 130 from the number of red pixels having a pixel value of 130 is a positive value, controller 52 uses the value as it is for calculation. If the value is a negative value, the value is considered zero. Next, controller 52 performs similar processing on each of the pixel values ranging from 131 to 255, inclusive. For instance, when there are 100 red pixels having a pixel value of 130 and 50 green pixels having a pixel value of 130, the value at a pixel value of 130 indicates 50. Moreover, for instance, when there are 100 red pixels having a pixel value of 131 and 150 green pixels having a pixel value of 131, the value at a pixel value of 131 indicates zero.

Moreover, in using the second method, for instance, controller 52 uses, for calculation, a value (the number of pixels) calculated by subtracting the number of pixels having green pixel values less than or equal to 129 (that is, the fourth pixel value is 129) from the number of pixels having red pixel values greater than or equal to 130 (that is, the first pixel value is 130). For instance, when there are 100 pixels whose red pixel values are greater than or equal to 130 and 50 pixels whose green pixel values are less than or equal to 129, the value indicates 50. Moreover, for instance, when there are 50 pixels whose red pixel values are greater than or equal to 130 and 100 pixels whose green pixel values are less than or equal to 129, the value indicates zero.

When the following Expression 1 using the first integrated value obtained by integrating positive values among the values is satisfied, controller 52 determines that external light is present.

$First integrated value / total number of pixels > 0.45$

In the above first method, the first integrated value for the two pixel values: a pixel value of 130 and a pixel value of 131 is 50 (50 pixels + zero pixels). Moreover, in the above second method, when there are 100 pixels whose red pixel values are greater than or equal to 130 and 50 pixels whose green pixel values are less than or equal to 129, the first integrated value is 50 (100 pixels - 50 pixels).

It should be noted that when the first integrated value is higher than a predetermined value, controller 52 may determine that external light is present. That is, whether external light is present may be determined without dividing the first integrated value by the total number of pixels. A value of 0.45 is an example of the first predetermined value.

Moreover, controller 52 determines that external light is present when the following Expression 2 using a third integrated value is satisfied, the third integrated value indicating the number of pixels having Value ranging from 250 to 255, inclusive.

$Third integrated value / total number of pixels > 0.45$

It should be noted that when the third integrated value is higher than a predetermined value, controller 52 may determine that external light is present. That is, whether external light is present may be determined without dividing the third integrated value by the total number of pixels. A value of 0.45 is an example of the third predetermined value.

Moreover, controller 52 may determine whether external light is entering on the basis of a second red pixel count including, for each of pixel values greater than or equal to a second pixel value higher than the first pixel value, a third pixel count indicating the number of red pixels having the pixel value. Moreover, in the embodiment, controller 52 determines whether external light is entering further on the basis of the first green pixel count including, for each of the pixel values greater than or equal to the first pixel value, the second pixel count indicating the number of green pixels having the pixel value.

In each of the pixel values greater than or equal to the second pixel value, controller 52 subtracts the second pixel count from the third pixel count, integrates one or more positive second subtraction values among second subtraction values each of which is calculated by subtracting the second pixel count from the third pixel count, and calculates a second incident level on the basis of a second integrated value calculated by integrating the one or more positive second subtraction values. For instance, controller 52 may determine, as a second incident level, a value calculated by dividing the second integrated value by the total number of pixels. Then, controller 52 determines whether the second incident level is lower than or equal to a second predetermined value, and outputs a determination result to display controller 53. In this way, controller 52 changes the display mode of the dental plaque detection result according to the determination result. It should be noted that the second pixel value is appropriately set according to the lighting environment of the space in which to use intraoral camera 10. For instance, when illuminance is 800 lux and the pixel value is expressed as 8 bits, the second pixel value may be 250.

FIG. 5 is a second figure with the plotted results of determination as to whether external light is present according to the embodiment. The horizontal axis in FIG. 5 indicates the second incident level (indicating "R-G Range 2" in FIG. 5), and the vertical axis indicates the third incident level ("V range" in FIG. 5). Moreover, in FIG. 5, the circles with the hatching pattern indicate the second incident level and the third incident level obtained from an image captured when external light was present. Meanwhile, the circles without the hatching pattern indicate the second incident level and the third incident level obtained from an image captured without external light.

Area E in FIG. 5 is the area in which all of the plotted circles are circles indicating the presence of external light. As such, by determining that external light is present when the second incident level is greater than or equal to threshold r3 (for example, 0.2), it is possible to determine with high accuracy that external light is present. In this case, since there is a high possibility of detecting a dental plaque area by error due to the external light, the dental plaque detection processing will not be performed. Threshold r3 is an example of the first predetermined value.

Area D in FIG. 5 is the area where both circles indicating the presence of external light and circles indicating the absence of external light are plotted. In this case, although the dental plaque detection processing is performed, there is a possibility of detecting a dental plaque area by error due to the external light. Thus, when the area where the second incident level and the third incident level are plotted is area D, the dental plaque detection result is displayed in the display mode that notifies the user that a dental plaque area may have been detected by error.

It should be noted that from the results shown in FIG. 5, when for instance the following determination expression is satisfied, controller 52 may determine that external light is present.

For instance, when a value (the number of pixels) calculated by subtracting the number of green pixels having a pixel value of 250 from the number of red pixels having a pixel value of 250 is a positive value, controller 52 uses the value as it is for calculation. When the value is a negative value, the value is considered zero. Next, controller 52 performs similar processing on each of the pixel values ranging from 251 to 255, inclusive. When the following Expression 3 using the second integrated value calculated by integrating one or more positive values among the values is satisfied, controller 52 determines that external light is present.

$Second integrated value / total number of pixels > 0.2$

It should be noted that when the second integrated value is higher than a predetermined value, controller 52 may determine that external light is present. That is, whether external light is present may be determined without dividing the second integrated value by the total number of pixels. A value of 0.2 is an example of the second predetermined value.

Controller 52 may perform, for example, determination for each of (Expression 1) to (Expression 3), and when at least one of the expressions is satisfied, controller 52 may determine that external light is present. For example, for the case of local exposure to external light, it may be determined that external light is not present in determination using Expression 1. However, by performing determination using Expression 2, even for the case of local exposure to external light, it is possible to increase the possibility of leading to the determination that external light is present. This makes it possible to determine with higher accuracy whether external light is present.

It should be noted that as described above, the pixel values at which to count pixels may be changed according to the lighting environment of the space. The pixel values (the first pixel value to the third pixel value described above) may be input by the user, for example.

FIG. 6 illustrates setting screen S1 of determination about external light according to the embodiment. Setting screen S1 is displayed on the touch screen.

As illustrated in FIG. 6, a pixel count range for counting pixels to determine whether external light is present may be settable by the user. Moreover, the thresholds for determining whether external light is present (0.45 in Expressions 1 and 2 and 0.2 in Expression 3) may be settable by the user.

It should be noted that portable terminal 50 stores a table in which the lighting environment in which to use the intraoral camera is associated with the settable pixel count range and thresholds in FIG. 6. The pixel count range and the thresholds may be automatically changed according to the table and the illuminance measured by sensor 22.

Moreover, in the dental plaque detection processing, controller 52 distinguishes teeth or dental plaque from lips or gums according to colors on an RGB image. It is known that when teeth are irradiated with light including a blue-light wavelength range, dentine emits excitation fluorescence, which transmits through an enamel layer and emits green fluorescence. Meanwhile, lips and gums take on a blue tinge, which enables controller 52 to readily distinguish teeth and dental plaque from lips and gums. Moreover, using an RGB image that has undergone the image processing such as the white balance adjustment processing makes it much easier to distinguish a dental plaque area from the other area. When a tooth area is made practically colorless by the image processing, it is easier to distinguish the dental plaque area that is the portion of a tooth to which dental plaque is adhering. Accordingly, controller 52 can readily identify the dental plaque area in a tooth image. Thus, for example, by capturing an image of a brushed tooth and identifying a dental plaque area, it is possible to present, to the user, the area that is not sufficiently brushed.

FIG. 2 is referenced again. Display controller 53 performs control to display the detected dental plaque area on touch screen 54. Display controller 53 changes the display mode of the dental plaque detection result according to whether the incident level of the external light which has entered the imaging area of an RGB image is lower than or equal to a predetermined value.

Touch screen 54 functions as an input device and an output device, and is configured to be able to display, for example, a dentition image. For instance, touch screen 54 may include a display such as a liquid crystal display panel and a touch panel laid on the display.

Here, an example of an image to be displayed on touch screen 54 is described with reference to FIG. 7. FIG. 7 illustrates captured images and display examples of dental plaque detection results for cases with and without the presence of external light according to the embodiment. FIG. 7 illustrates a captured image and a display example of the dental plaque detection result in each of the cases with and without the presence of external light. The captured image is an RGB image obtained by intraoral camera 10 capturing an image of teeth and gums inside the oral cavity. Moreover, an image displaying the dental plaque detection result is an image in which the dental plaque area detected after the dental plaque detection processing of controller 52 is highlighted, and is an image displayed on touch screen 54.

For the case without external light, the dental plaque area shown in the captured image and the area determined to have dental plaque in the image displaying the dental plaque detection result are almost identical areas.

Meanwhile, for the case with the presence of external light, although the captured image does not include a dental plaque area, the image displaying the dental plaque detection result shows the area determined to have dental plaque. The area is a false positive area and the area detected by error due to the effects of the external light. In this case, the user may be notified that the area is the false positive area (see, for example, FIG. 9 described later).

As such, by using the intraoral camera system, the user can capture an image of their oral cavity with intraoral camera 10 and check the intraoral condition displayed on portable terminal 50. This enables the user to readily check their teeth health condition, for example.

### [2. Operation of Intraoral Camera System]

Next, the operation of the intraoral camera system configured as described above is described with reference to FIGS. 8 to 10. FIG. 8 is a flowchart illustrating an operation (image processing method) of portable terminal 50 according to the embodiment. It should be noted that the processing illustrated in FIG. 8 is, for example, processing performed in real time, and is performed per frame or every time image data including a plurality of frames is obtained.

Intraoral camera 10 transmits, to portable terminal 50, image data (an RGB image) generated by the user capturing an image of teeth and gums inside their oral cavity with the use of intraoral camera 10. Here, the image data may be a video, one still image, or two or more still images. Moreover, when the image data is a video or includes two or more still images, the sensor data (external light detection result) of sensor 22 may be transmitted for each frame of the video or for each still image. It should be noted that when the image data is a video, sensor data may be transmitted every two or more frames.

Moreover, the image data may be transmitted in real time or transmitted at once after a series of image capturing (e.g., after capturing images of all the teeth inside the oral cavity).

As illustrated in FIG. 8, obtainer 51 obtains an RGB image transmitted from intraoral camera 10 (S11). The RGB image is an image obtained by intraoral camera 10 capturing an image of teeth and dental plaque fluorescing in response to exposure to light including the blue-light wavelength range.

Then, controller 52 obtains the incident level of external light on the basis of the obtained RGB image (S12). Controller 52 may calculate the incident level by, for example, dividing at least one of the first to third integrated values based on the RGB image by the total number of pixels. It should be noted that the incident level includes at least one of the first to third incident levels.

Then, controller 52 performs the dental plaque detection processing on the basis of the obtained RGB image (S13).

Then, controller 52 determines whether the incident level obtained in step S12 is lower than or equal to the predetermined value (S14). Step S14 is equivalent to determination as to whether external light is entering the oral cavity.

When determining that the incident level is not lower than or equal to the predetermined value (No in S14), controller 52 outputs a determination result obtained in step S14 to display controller 53, thereby causing touch screen 54 to display information indicating that external light is entering and an image including a dental plaque detection result or an image not including the dental plaque detection result (for example, the RGB image obtained from intraoral camera 10) (S15). Moreover, when determining that the incident level is lower than or equal to the predetermined value (Yes in S14), controller 52 outputs a determination result obtained in step S14 to display controller 53, thereby causing touch screen 54 to display an image including the dental plaque detection result (perform normal display) (S16).

FIG. 9 illustrates display examples indicating the presence and absence of external light according to the embodiment. FIG. 9 illustrates dentition images. It should be noted that in (a) and (b) in FIG. 9, illustration of display (for example, highlighted display) of a dental plaque detection result is omitted for the sake of convenience.

(a) in FIG. 9 is the image displayed in step S15, and first information (the circle with the diagonal line hatching shown in (a) in FIG. 9) superposed on the image including the dental plaque detection result is displayed. Here, the first information indicates that external light is entering (or there is a possibility that external light is entering). The first information superposed on, for example, an area different from a teeth area and a dental plaque area (for example, the highlighted display area) is displayed. It should be noted that when the incident level is not lower than or equal to the predetermined value, controller 52 may prohibit the displaying of the dental plaque detection result or the performing of the dental plaque detection processing.

(b) in FIG. 9 is the image displayed in step S16, and second information (the circle without a diagonal line hatching shown in (b) in FIG. 9) superposed on the image including the dental plaque detection result is displayed. Here, the second information indicates that external light is not entering. The second information is information different from the first information. It should be noted that the second information need not be displayed. That is, for step S16, an image including only the dental plaque detection result out of the information indicating that light is not entering and the dental plaque detection result may be displayed.

In this way, for the case with the presence of external light, controller 52 causes the first information to be displayed. For the case without external light, controller 52 causes the second information or only the dental plaque detection result to be displayed. In this way, controller 52 changes the display mode of the dental plaque detection result according to whether external light is present.

It should be noted that controller 52 may further determine whether at least one of the following conditions is satisfied. The conditions are (i) the first incident level is lower than or equal to the first predetermined value (for example, threshold r1 illustrated in FIG. 4) and greater than or equal to the fourth predetermined value (for example, threshold r2 illustrated in FIG. 4) lower than the first predetermined value and (ii) the third incident level is lower than or equal to the third predetermined value (for example, threshold v1 illustrated in FIG. 4) and greater than and equal to the fifth predetermined value (for example, threshold v2 illustrated in FIG. 4) lower than the third predetermined value. When determining that at least one of the conditions is satisfied, by outputting the determination result to display controller 53, controller 52 may cause the touch screen to display information suggesting blocking light and superposed on an image displaying the dental plaque detection result. It should be noted that each predetermined value may be set according to the lighting environment in which an RGB image is to be captured.

FIG. 10 illustrates an example of displayed information suggesting blocking light according to the embodiment.

As illustrated in FIG. 10, controller 52 may provide a notification suggesting the user prevent external light from entering the oral cavity by blocking light. Moreover, controller 52 may cause the touch screen to display information suggesting dimming the light or turning off the light, instead of the information suggesting blocking light. It should be noted that the information suggesting blocking light may include, for example, information suggesting closing the curtains. It should be noted that the information suggesting blocking light is superposed on an area different from a teeth area and a dental plaque area (for example, the highlighted display area), and the superposed information is displayed.

### (Variation 1 of Embodiment)

Hereinafter, an intraoral camera system according to Variation 1 of the embodiment is described with reference to FIG. 11. It should be noted that hereinafter, the differences from the embodiment are focused on, and the explanations of the same or similar points in the variation and the embodiment are omitted or simplified.

FIG. 11 schematically illustrates a state where a captured image is divided into a plurality of areas according to Variation 1.

As illustrated in FIG. 11, controller 52 may divide the RGB image into a plurality of areas (the four areas: areas R1 to R4 in the example illustrated in FIG. 11), and obtain the incident level of external light entering each of the divided plurality of areas. Controller 52 may change the display mode of a dental plaque detection result according to the result of determination as to whether the incident level in each of the plurality of areas is lower than or equal to the predetermined value. For instance, when the incident level in at least one of the plurality of areas is not lower than or equal to the predetermined value, controller 52 may determine that external light is present. Controller 52 may perform, for each of the plurality of areas, the determination in each of Expressions 1 and 3 indicated in the embodiment, and when at least one of Expression 1 or 3 is satisfied in at least one of the plurality of areas, controller 52 may determine that external light is present.

It should be noted that, for example, a value larger than the first pixel value in the embodiment is set to a first pixel value. For instance, the smaller the area on the image for which the incident level is determined, a larger first pixel value may be set. Moreover, as with the first pixel value, for example, a value larger than the second pixel value in the embodiment is set to a second pixel value. In Variation 1, the first pixel value is 0.8 as a non-limiting example, and the second pixel value is 0.5 as a non-limiting example. Intraoral camera 10 stores a table in which the sizes of the plurality of areas are associated with the first pixel value and the second pixel value, and the first pixel value and the second pixel value may be automatically set according to the table. Moreover, for example, the sizes of the plurality of areas may be set by the user.

It should be noted that the number of the plurality of areas into which the image is divided is not limited to a particular number as long as the number of divided areas is at least two. Moreover, for example, the sizes and the shapes of the plurality of areas may be the same or different.

### (Variation 2 of Embodiment)

Hereinafter, an intraoral camera system according to Variation 2 of the embodiment is described with reference to FIGS. 14 to 24. It should be noted that hereinafter, the differences from the embodiment are focused on, and the explanations of the same or similar points in the variation and the embodiment are omitted or simplified.

FIG. 14 is a flowchart illustrating an operation (image processing method) of portable terminal 50 according to Variation 2. The intraoral camera system according to Variation 2 divides an RGB image into a plurality of areas and presents, to a user, information suggesting solution to external light, in accordance with the incident level of external light in each of the plurality of areas (for example, the presence or absence of external light). In this respect, the intraoral camera system according to Variation 2 differs from the intraoral camera system according to the embodiment. The reference sings used in the intraoral camera system according to the embodiment are used in the following descriptions.

As illustrated in FIG. 14, obtainer 51 obtains an RGB image transmitted from intraoral camera 10 (S21). The RGB image is an image obtained by intraoral camera 10 capturing an image of teeth and dental plaque fluorescing in response to exposure to light including a blue-light wavelength range.

Then, controller 52 divides the RGB image into a plurality of areas (S22). Controller 52 may divide the RGB image into two or more areas. For instance, as illustrated in FIG. 11, the RGB image may be divided into four areas. Alternatively, the RGB image may be divided into nine areas or another number of areas. In Variation 2, cases where the RGB image is divided into four areas and cases where the RGB image is divided into nine areas are mainly described.

It should be noted that hereinafter, an instance in which the plurality of areas are quadrangular areas is described. However, the shapes are not limited to quadrangular shapes. For instance, the plurality of areas may be in any other shapes including, for example, a circle, a triangle, and a polygon with five or more sides. Moreover, one RGB image may include areas with different shapes. Moreover, the areas of the plurality of areas may be equal, and the area of at least one area may differ from the area of the other areas. It should be noted that a quadrangle may be a square or a rectangle.

Next, controller 52 obtains the incident level of external light entering each of the plurality of areas (S23). Controller 52 calculates, for instance, at least one of the first to third integrated values for each of the plurality of areas, and divides the at least one integrated value calculated, by the total number of pixels in the area, thereby calculating the incident level. An incident level is calculated per area. It should be noted that obtainment of the incident level of each of the plurality of areas is not limited to the obtainment through calculation, and the incident level may be obtained from an external device via obtainer 51.

Then, controller 52 determines whether external light is present for each of the plurality of areas (S24). Controller 52 determines whether external light is present for each of the plurality of areas according to the incident level of the area. For instance, using the above (Expression 1) or (Expression 2), controller 52 may determine whether external light is present for each of the plurality of areas. In this case, the first integrated value indicated in (Expression 1) and the second integrated value indicated in (Expression 2) are values in the area, and the total number of pixels is the total number of pixels included in the area.

Then, controller 52 determines the incident direction of external light relative to the RGB image (S25). That is, controller 52 determines the incident direction of external light entering the imaging area inside the oral cavity. For instance, controller 52 may determine the incident direction of external light according to the positional relationship (for example, the positions in the RGB image) of one or more areas in which the incident level exceeds a predetermined value (that is, one or more areas in which it is determined that external light is present) among the plurality of areas.

It should be noted that controller 52 determines whether external light is entering the RGB image in accordance with a determination result obtained in step S24. When determining that external light is entering the RGB image, controller 52 may perform the processing from step S25, and when determining that external light is not entering the RGB image, controller 52 may end the processing. When determining that external light is present in at least one of the plurality of areas in step S24, controller 52 may determine that external light is entering the RGB image.

Here, examples of a relationship between the positions of one or more areas in which it is determined that external light is present in the RGB image and the incident direction of the external light are described with reference to FIGS. 15A to 21H. First, the cases where the plurality of areas are four areas (the RGB image is divided into four areas) are described with reference to FIGS. 15A to 16D. By dividing the RGB image into four areas, it is possible to determine the incident direction of external light while suppressing an increase in the processing of portable terminal 50, for instance. FIGS. 15A to 15H illustrate examples of the determination results of incident levels in the four divided areas in the cases where external light enters in respective directions. FIGS. 16A to 16D illustrate other examples of the determination results of incident levels in the four divided areas in the cases where external light enters in respective directions. Moreover, an area in which it is determined that external light is present is indicated in the diagonal line hatching, and an area in which it is determined that external light is not present is indicated in a square frame (without hatching).

FIGS. 15A to 15D each illustrate the incident direction of external light when it is determined that external light is present in one of the four divided areas. FIGS. 15E to 15H each illustrate the incident direction of external light when it is determined that external light is present in two of the four divided areas. FIGS. 16A to 16D each illustrate the incident direction of external light when it is determined that external light is present in three of the four divided areas. It should be noted that the four divided areas are referred to as areas R11 to R14. Area R11 is the upper left area in the RGB image. Area R12 is the upper right area in the RGB image. Area R13 is the lower left area in the RGB image. Area R14 is the lower right area in the RGB image.

As illustrated in FIGS. 15A and 15B, when it is determined that external light is present only in area R11, controller 52 determines that external light is entering from the upper left (the upper left side) of the RGB image, and when it is determined that external light is present only in area R12, controller 52 determines that external light is entering from the upper right (the upper right side) of the RGB image. Moreover, as illustrated in FIGS. 15C and 15D, when it is determined that external light is present only in area R14, controller 52 determines that external light is entering from the lower right (the lower right side) of the RGB image, and when it is determined that external light is present only in area R13, controller 52 determines that external light is entering from the lower left (the lower left side) of the RGB image. As such, when it is determined that external light is present only in one of the four divided areas, controller 52 may determine, for instance, that the external light is entering in one of oblique directions.

Moreover, as illustrated in FIGS. 15E and 15F, when it is determined that external light is present only in areas R11 and R12, controller 52 determines that external light is entering from the upper portion (the upper side) of the RGB image, and when it is determined that external light is present only in areas R12 and R14, controller 52 determines that external light is entering from the right (the right side) of the RGB image. Moreover, as illustrated in FIGS. 15G and 15H, when it is determined that external light is present only in areas R13 and R14, controller 52 determines that external light is entering from the lower portion (the lower side) of the RGB image, and when it is determined that external light is present only in areas R11 and R13, controller 52 determines that external light is entering from the left (the left side) of the RGB image. As such, when it is determined that external light is present only in two of the four divided areas, controller 52 may determine, for instance, that the external light is entering from the upper side, from the lower side, from the left, or from the right. It should be noted that the two areas in which it is determined that external light is present may be, for example, diagonally positioned areas in the RGB image. For instance, it may be determined that external light is present only in areas R11 and R14 or only in areas R12 and R13. In this case, controller 52 may determine that external light is entering in two directions, for instance.

Moreover, as illustrated in FIGS. 16A and 16B, when it is determined that external light is present only in areas R11 to R13, controller 52 determines that external light is entering from the upper left of the RGB image, and when it is determined that external light is present only in areas R11, R12, and R14, controller 52 determines that external light is entering from the upper right of the RGB image. Moreover, as illustrated in FIGS. 16C and 16D, when it is determined that external light is present only in areas R12 to R14, controller 52 determines that external light is entering from the lower right of the RGB image, and when it is determined that external light is present only in areas R11, R13, and R14, controller 52 determines that external light is entering from the lower left of the RGB image. As such, when it is determined that external light is present only in three of the four divided areas, controller 52 may determine, for instance, that the external light is entering in one of the oblique directions.

Next, the cases where the plurality of areas are nine areas (the RGB image is divided into nine areas) are described with reference to FIGS. 17A to 21H. By dividing the RGB image into nine areas, it is possible to determine the incident direction of external light more precisely, for example. FIGS. 17A to 21H illustrate examples of the determination results of incident levels in the nine divided areas in the cases where external light enters in respective directions. FIGS. 17A to 17H each illustrate the incident direction of external light when it is determined that external light is present in one of the nine divided areas. FIGS. 18A to 18H each illustrate the incident direction of external light when it is determined that external light is present in two of the nine divided areas. FIGS. 19A to 19H each illustrate the incident direction of external light when it is determined that external light is present in three of the nine divided areas. Moreover, FIGS. 20A to 20H each illustrate the incident direction of external light when it is determined that external light is present in four of the nine divided areas. FIGS. 21A to 21H each illustrate the incident direction of external light when it is determined that external light is present in five of the nine divided areas.

It should be noted that the nine divided areas are referred to as areas R21 to R29. Area R21 is the upper left portion of the RGB image. Area R22 is the upper middle portion of the RGB image. Area R23 is the upper right portion of the RGB image. Area R24 is the middle left portion of the RGB image. Area R25 is the middle portion of the RGB image. Moreover, area R26 is the middle right portion of the RGB image. Area R27 is the lower left portion of the RGB image. Area R28 is the lower middle portion of the RGB image. Area R29 is the lower right portion of the RGB image.

As illustrated in FIGS. 17A and 17B, when it is determined that external light is present only in area R21, controller 52 determines that external light is entering from the upper left of the RGB image, and when it is determined that external light is present only in area R23, controller 52 determines that external light is entering from the upper right of the RGB image. Moreover, as illustrated in FIGS. 17C and 17D, when it is determined that external light is present only in area R27, controller 52 determines that external light is entering from the lower left of the RGB image, and when it is determined that external light is present only in area R29, controller 52 determines that external light is entering from the lower right of the RGB image.

As illustrated in FIGS. 17E and 17F, when it is determined that external light is present only in area R24, controller 52 determines that external light is entering from the left of the RGB image, and when it is determined that external light is present only in area R22, controller 52 determines that external light is entering from the upper portion of the RGB image. Moreover, as illustrated in FIGS. 17G and 17H, when it is determined that external light is present only in area R28, controller 52 determines that external light is entering from the lower portion of the RGB image, and when it is determined that external light is present only in area R26, controller 52 determines that external light is entering from the right of the RGB image.

FIGS. 18A to 18D each illustrate a case where it is determined that external light is present in two vertically consecutive areas, and FIGS. 18E to 18H each illustrate a case where it is determined that external light is present in two horizontally consecutive areas.

As illustrated in FIGS. 18A and 18B, when it is determined that external light is present only in areas R21 and R24, controller 52 determines that external light is entering from the upper left of the RGB image, and when it is determined that external light is present only in areas R23 and R26, controller 52 determines that external light is entering from the upper right of the RGB image. Moreover, as illustrated in FIGS. 18C and 18D, when it is determined that external light is present only in areas R24 and R27, controller 52 determines that external light is entering from the lower left of the RGB image, and when it is determined that external light is present only in areas R26 and R29, controller 52 determines that external light is entering from the lower right of the RGB image.

As illustrated in FIGS. 18E and 18F, when it is determined that external light is present only in areas R21 and R22, controller 52 determines that external light is entering from the upper left of the RGB image, and when it is determined that external light is present only in areas R22 and R23, controller 52 determines that external light is entering from the upper right of the RGB image. Moreover, as illustrated in FIGS. 18G and 18H, when it is determined that external light is present only in areas R27 and R28, controller 52 determines that external light is entering from the lower left of the RGB image, and when it is determined that external light is present only in areas R28 and R29, controller 52 determines that external light is entering from the lower right of the RGB image.

It should be noted that the two consecutive areas may be areas including area R25 and diagonally consecutive areas (for example, areas R23 and R25).

FIGS. 19A to 19D each illustrate a case where it is determined that external light is present in three areas arranged in an L-shape, and FIGS. 19E to 19H each illustrate a case where it is determined that external light is present in three areas consecutively aligned in a straight line.

As illustrated in FIGS. 19A and 19B, when it is determined that external light is present only in areas R21, R22, and R24, controller 52 determines that external light is entering from the upper left of the RGB image, and when it is determined that external light is present only in areas R22, R23, and R26, controller 52 determines that external light is entering from the upper right of the RGB image. Moreover, as illustrated in FIGS. 19C and 19D, when it is determined that external light is present only in areas R24, R27, and R28, controller 52 determines that external light is entering from the lower left of the RGB image, and when it is determined that external light is present only in areas R26, R28, and R29, controller 52 determines that external light is entering from the lower right of the RGB image. As such, when it is determined that external light is present only in the three areas arranged in an L-shape out of the nine divided areas, controller 52 may determine, for instance, that the external light is entering in one of the oblique directions.

As illustrated in FIGS. 19E and 19F, when it is determined that external light is present only in areas R21, R24, and R27, controller 52 determines that external light is entering from the left of the RGB image, and when it is determined that external light is present only in areas R21 to R23, controller 52 determines that external light is entering from the upper portion of the RGB image. Moreover, as illustrated in FIGS. 19G and 19H, when it is determined that external light is present only in areas R27 to R29, controller 52 determines that external light is entering from the lower portion of the RGB image, and when it is determined that external light is present only in areas R23, R26, and R29, controller 52 determines that external light is entering from the right of the RGB image. As such, when it is determined that external light is present only in the three areas aligned in a straight line out of the nine divided areas, controller 52 may determine, for instance, that the external light is entering from the upper side, from the lower side, from the left, or from the right.

FIGS. 20A to 20D each illustrate a case where it is determined that external light is present in four areas arranged in a square shape, and FIGS. 20E to 20H each illustrate a case where it is determined that external light is present in four areas arranged in an L-shape.

As illustrated in FIGS. 20A and 20B, when it is determined that external light is present only in areas R21, R22, R24 and R25, controller 52 determines that external light is entering from the upper left of the RGB image, and when it is determined that external light is present only in areas R22, R23, R25, and R26, controller 52 determines that external light is entering from the upper right of the RGB image. Moreover, as illustrated in FIGS. 20C and 20D, when it is determined that external light is present only in areas R24, R25, R27, and R28, controller 52 determines that external light is entering from the lower left of the RGB image, and when it is determined that external light is present only in areas R25, R26, R28, and R29, controller 52 determines that external light is entering from the lower right of the RGB image. As such, when it is determined that external light is present only in the four areas arranged in a square shape out of the nine divided areas, controller 52 may determine, for instance, that the external light is entering in one of the oblique directions.

As illustrated in FIGS. 20E and 20F, when it is determined that external light is present only in areas R21 to R24, controller 52 determines that external light is entering from the upper left of the RGB image, and when it is determined that external light is present only in areas R21 to R23 and area R26, controller 52 determines that external light is entering from the upper right of the RGB image. Moreover, as illustrated in FIGS. 20G and 20H, when it is determined that external light is present only in area R24 and areas R27 to R29, controller 52 determines that external light is entering from the lower left of the RGB image, and when it is determined that external light is present only in areas R26 to R29, controller 52 determines that external light is entering from the lower right of the RGB image. As such, when it is determined that external light is present only in the four areas arranged in an L-shape out of the nine divided areas, controller 52 may determine, for instance, that the external light is entering in one of the oblique directions.

FIGS. 21A to 21D each illustrate a case where it is determined that external light is present in five areas side by side that include central area R25. FIGS. 21E to 21H each illustrate a case where it is determined that external light is present in five areas side by side that do not include central area R25 (for example, areas arranged in a U-shape).

As illustrated in FIGS. 21A and 21B, when it is determined that external light is present only in areas R21 to R25, controller 52 determines that external light is entering from the upper left of the RGB image, and when it is determined that external light is present only in areas R21 to R23 and areas R25 and R26, controller 52 determines that external light is entering from the upper right of the RGB image. Moreover, as illustrated in FIGS. 21C and 21D, when it is determined that external light is present only in areas R21, R24, R25, R27, and R28, controller 52 determines that external light is entering from the lower left of the RGB image, and when it is determined that external light is present only in areas R23, R25, R26, R28, and R29, controller 52 determines that external light is entering from the lower right of the RGB image. As such, when it is determined that external light is present only in the five side-by-side areas including central area R25 out of the nine divided areas, controller 52 may determine, for instance, that the external light is entering in one of the oblique directions.

As illustrated in FIGS. 21E and 21F, when it is determined that external light is present only in areas R21 to R24 and area R26, controller 52 determines that external light is entering from the upper portion of the RGB image, and when it is determined that external light is present only in area R24 and areas R26 to R29, controller 52 determines that external light is entering from the lower portion of the RGB image. Moreover, as illustrated in FIGS. 21G and 21H, when it is determined that external light is present only in areas R21, R22, R24, R27, and R28, controller 52 determines that external light is entering from the left of the RGB image, and when it is determined that external light is present only in areas R22, R23, R26, R28, and R29, controller 52 determines that external light is entering from the right of the RGB image. As such, when it is determined that external light is present only in the five areas arranged in a U-shape out of the nine divided areas or in the five areas arranged in a U-shape rotated by 90 degrees, in a U-shape rotated by 180 degrees, or in a U-shape rotated by 270 degrees, controller 52 may determine, for instance, that the external light is entering in from the upper side, from the lower side, from the left, or from the right.

It should be noted that the exemplified positions of the areas in which it is determined that external light is present in FIGS. 15A to 21H are examples and are not limited to the above positions. Moreover, the exemplified correspondences between the incident directions of external light and the areas in which it is determined that external light is present in FIGS. 15A to 21H are examples and are not limited to the above-mentioned correspondences. For instance, two or more incident directions of external light may be determined according to at least one of the number or positions of areas in which it is determined that external light is present.

It should be noted that it is possible to identify the direction(s) in which external light is not entering, from the result of determination as to whether external light is present obtained for each of the plurality of areas in FIGS. 15A to 21H. The direction(s) in which external light is not entering may be used in, for example, determining solution information, which is described later.

FIG. 14 is referenced again. Then, portable terminal 50 outputs solution information that depends on the incident direction (S26). For instance, controller 52 may determine solution information including a solution based on the incident direction, and transmit the determined solution information to an external device. Moreover, display controller 53 may cause touch screen 54 to display the solution information determined by controller 52.

Here, the determined solution information is described with reference to FIGS. 22 to 24. FIGS. 22 to 24 illustrate an example of each solution information according to Variation 2. As a non-limiting example, the second solution information illustrated in FIG. 23 includes a solution that places a larger performance burden on a user than that included in the first solution information illustrated in FIG. 22. As a non-limiting example, the third solution information illustrated in FIG. 24 includes a solution that places a larger burden on the user than that included in the second solution information illustrated in FIG. 23. It should be noted that examples of a large performance burden include an increase in the parts of body the user moves and an increase in the moving area of the user themselves (for example, this includes a change from a solution that does not require the movement of the user themselves from the original location to a solution that requires the movement of the user themselves from the original location).

It should be noted that FIG. 22 illustrates a solution when external light is entering from above. FIG. 23 illustrates a solution when external light is entering from the upper right. FIG. 24 illustrates a solution when there are many areas in which it is determined that external light is present.

FIG. 22 illustrates a display example of the first solution information including, as a solution, wearing a light shielding cover or bending the head down. To bend the head down is equivalent to turn the face away from external light. As such, a solution suggesting turning the head to face an appropriate direction based on the incident direction of external light may be presented. Moreover, information indicating that a care condition cannot be properly evaluated is displayed. To display the information is an example of notifying that a dental plaque area may have been detected by error due to the external light that entered the oral cavity. In this way, it is possible to suggest the user perform the solution included in the first solution information. It should be noted that to bend the head down is an example of information regarding the incident direction of external light.

FIG. 23 illustrates a display example of the second solution information including, as a solution, covering the mouth from the upper right with a hand not holding a toothbrush while keeping the head down. The solution illustrated in FIG. 23 includes covering the mouth with the hand to block the incident direction of external light, in addition to bending the head down illustrated in FIG. 22. As such, a solution suggesting covering the mouth with the hand to block an appropriate direction based on the incident direction of external light.

For instance, when it is determined that external light is present in one area or two areas out of the four areas into which the RGB image has been divided (see FIGS. 15A to 15H) or when it is determined that external light is present in one to four areas out of the nine areas into which the RGB image has been divided (see FIGS. 17A to 20H), controller 52 may determine that the first solution information or the second solution information will be output. For instance, when it is determined that external light is present in one of the four areas into which the RGB image has been divided (see FIGS. 15A to 15D) or when it is determined that external light is present in one or two areas out of the nine areas into which the RGB image has been divided (see FIGS. 17A to 18H), controller 52 may determine that the first solution information will be output. Moreover, for instance, when it is determined that external light is present in two areas out of the four areas into which the RGB image has been divided (see FIGS. 15E to 15H) or when it is determined that external light is present in three or four areas out of the nine areas into which the RGB image has been divided (see FIGS. 19A to 20H), controller 52 may determine that the second solution information will be output.

Moreover, for instance, when it is determined that external light is present in one or two areas out of the four areas into which the RGB image has been divided or when it is determined that external light is present in one to four areas in the nine areas into which the RGB image has been divided, if the determination that external light is present is the first time determination, controller 52 may select the first solution information, and if the determination is the second time determination (that is, although solution information was output in response to the first time determination and the solution thereof was performed, it is determined that external light is still present), controller 52 may select the second solution information. Thus, controller 52 may determine the solution information to be output, according to whether it is determined that external light is present after the solution information was output.

FIG. 24 illustrates a display example of the third solution information including, as a solution, turning off the light in the room or changing the place in which to use an intraoral camera. The first solution information and the second solution information are solutions feasible without the user themselves moving to another place. Meanwhile, the third solution information includes the following: the user themselves moves to another place or the user controls a device external to the intraoral camera system. Thus, the third solution information includes a solution that places a larger performance burden on the user than the solutions included in the first solution information and the second solution information.

For instance, when it is determined that external light is present in three or more areas out of the four areas into which the RGB image has been divided (see FIGS. 16A to 16D) or when it is determined that external light is present in five or more areas out of nine areas into which the RGB image has been divided (see FIGS. 21A to 21H), controller 52 may select the third solution information.

Moreover, for instance, when it is determined that external light is present in one or two areas out of the four areas into which the RGB image has been divided or when it is determined that external light is present in one to four areas out of the nine areas into which the RGB image has been divided, if the determination that external light is present is the determination made for the second or more times, controller 52 may select the third solution information. For instance, although the second solution information was output and the solution thereof was performed, if it is determined that external light is present, controller 52 may select the third solution information. Thus, controller 52 may determine the solution information according to whether it is determined that external light is present after the solution information was output.

Moreover, controller 52 may determine the solution information on the basis of the positions of one or more areas in which the incident level exceeds a predetermined value (that is, one or more areas in which it is determined that external light is present). For instance, when there is only one incident direction of external light, controller 52 may select the first solution information or the second solution information, and when there are two or more incident directions of external light, controller 52 may select the third solution information.

It should be noted that portable terminal 50 may determine the solution information corresponding to at least one of the number of one or more areas, among the plurality of areas, in which the incident level exceeds the predetermined value or the positions of the one or more areas (the positions on the RGB image), among the plurality of areas, in which the incident level exceeds the predetermined value, and display the determined solution information.

It should be noted that at least one of the first solution information to the third solution information may include information regarding the incident direction of external light. The information regarding the incident direction of external light may be information indicating in which direction external light is entering and may be information indicating a solution based on the incident direction of external light.

FIG. 14 is referenced again. Controller 52 determines whether the user has performed the solution indicated in the output solution information (S27). In Variation 2, controller 52 functions as a determiner that determines whether the user has responded to the output solution information. For instance, controller 52 may determine that the user has performed the solution included in the solution information when touch screen 54 has received user's input indicating that the user has performed the solution included in the solution information, when information that is based on the image of the user captured by an external imaging apparatus and indicates that the solution indicated in the solution information has been performed is obtained via obtainer 51, or when a predetermined period of time has elapsed since the solution information was output.

When determining that the user has performed the solution (Yes in S27), portable terminal 50 determines whether external light is blocked (S28). Specifically, portable terminal 50 performs the processing from steps S21 to S24 again. For instance, after the user is determined to have responded to the solution information, portable terminal 50 may obtain post-response incident levels in the plurality of areas in a second RGB image obtained by capturing an image of teeth and dental plaque fluorescing in response to exposure to light including a blue-light wavelength range.

In step S24 performed again, when it is determined for each of the plurality of areas that external light is not present, controller 52 selects Yes in step S28. When it is determined for at least one of the plurality of areas that external light is present, controller 52 selects No in step S28.

When the external light is not blocked (No in S28), controller 52 outputs solution information different from the solution information output in step S26 (S29). Another solution information is, for example, information based on at least one of the number of one or more areas, among the plurality of areas, in which the post-response incident level exceeds the predetermined value or the positions of the one or more areas among the plurality of areas.

When the first solution information was output in step S26, controller 52 may output the second solution information in step S29. When the second solution information was output in step S26, controller 52 may output the third solution information in step S29. Controller 52 may select the second solution information different from the first solution information according to the post-response incident levels in the plurality of areas, and cause the touch panel to display the second solution information.

Moreover, controller 52 may select another solution information according to a difference between at least one of the number of one or more areas in which it is determined in step S24 that external light is present or the positions of the one or more areas (that is, the incident direction(s) of external light) and at least one of the number of one or more areas in which it is determined in step S28 that external light is present or the positions of the one or more areas (that is, the incident direction(s) of external light). For instance, when at least one of (i) the condition that the first solution information was output in step S26 and the number of areas where external light is determined to be present in step S28 is greater than or equal to the number of areas where external light is determined to be present in step S24 or (ii) the condition that the incident direction of external light is the same in steps S28 and S24 is satisfied, controller 52 may output the third solution information as another solution information. When at least one of (i) the condition that the number of areas where external light is determined to present in step S28 is less than the number of areas where external light is determined to be present in step S24 or (ii) the condition that the incident direction of external light is different in steps S28 and S24, controller 52 may output the second solution information as another solution information.

It should be noted that the method of determining another solution information is not limited to the above method and another method may be used in determining. For instance, another solution information may be determined on the basis of the output solution information and a table in which at least one of the number or positions of areas in which it is determined that external light is present before and after the output of the solution information is associated with another solution information.

After step S29 is performed and when the determination result is No in step S27, the processing returns to S27, from where the processing will be performed.

Moreover, when external light is blocked (Yes in S28), controller 52 performs the processing from step S30.

The processing in step S30 is similar to that indicated in step S13 in FIG. 8, and the processing indicated in step S31 is similar to that indicated in step S16 in FIG. 8. Thus, the explanations of the processing indicated in those steps are omitted.

### [Other Embodiments]

The intraoral camera system(s) according to one aspect or aspects are described above on the basis of the embodiment and the variations thereof. However, the present disclosure is not limited to the embodiment and the variations thereof. The present disclosure may encompass embodiments obtained by making various changes envisioned by those skilled in the art to the embodiment and embodiments obtained by combining some of the constituent elements described in different embodiments as long as the resultant embodiments do not depart from the scope of the present disclosure.

For instance, in the above embodiment and variations thereof, portable terminal 50 is provided as an example of the information terminal of the user. However, the information terminal may be a stationary information terminal.

Moreover, intraoral camera 10 in the above embodiment and the variations thereof may be provided in, for example, an oral cleaning device.

Moreover, in the embodiment and variations thereof, the instance in which error detection is notified by displaying is described. However, a notification method is not limited to displaying. For instance, error detection may be notified by outputting sound or emitting light (for example, a warning light). In this case, portable terminal 50 may include a sound outputting apparatus such as a loudspeaker or a light emitting apparatus such as an LED.

Moreover, in the above embodiment and the variations thereof, the instance in which the imaging processing such as AWB is performed by image processor 32 of intraoral camera 10 is described. However, controller 52 of portable terminal 50 may perform the image processing, for example. Moreover, some or all of the processing tasks performed by controller 52 of portable terminal 50 may be performed by controller 33 of intraoral camera 10, for example.

Moreover, in Variation 2 of the above embodiment, as a non-limiting example, the instance in which solution information is determined and displayed in accordance with the result of determination as to whether external light is present in each of a plurality of areas is described. However, for instance, solution information to suppress the effects of external light may be displayed without dividing an RGB image into a plurality of areas. For instance, as described in the embodiment, controller 52 may display solution information that depends on an incident level obtained from the entire area of an RGB image. For instance, when the incident level is less than a first level, controller 52 selects the first solution information. When the incident level is at least the first level and less than a second level higher than the first level, controller 52 selects the second solution information. When the incident level is greater than or equal to the second level, controller 52 may select the third solution information.

Moreover, as non-limiting example, the instance is described in which portable terminal 50 according to the embodiment changes the display mode of a dental plaque detection result according to the incident level of external light that has entered the imaging area of an RGB image and displays solution information that depends on the incident level to suppress the external light. However, for instance, the present disclosure may be achieved as, for example, an image processing method in which solution information that depends on the incident level to suppress the external light is displayed without changing the display mode of a dental plaque detection result. For instance, the present disclosure may be achieved as an image processing method for displaying a dental plaque area on the basis of an RGB image obtained by capturing an image of teeth and dental plaque fluorescing in response to exposure to light including a blue-light wavelength range, the image processing method including: obtaining the incident level of external light entering the imaging area of the RGB image; and displaying solution information that depends on the incident light to suppress the effects of the external light. This makes it possible to effectively suppress external light entering the oral cavity.

Moreover, in Variation 2 of the above embodiment, although the instance of displaying solution information is described. However, for instance, an RGB image further reflecting the result of determination as to whether external light is present (for instance, an RGB image explicitly indicating the area in which the external light is present) may be displayed. In this case, a mirror reversed image of the RGB image may be displayed. In this way, the incident direction of the external light that a user sees in the image matches the actual incident direction, which makes it easier for the user to intuitively recognize the incident direction.

Moreover, in the above embodiment and the variation thereof, each of the constituent elements may be configured as dedicated hardware or may be achieved by executing a software program suitable for the constituent element. The constituent element may be achieved by a program executer, such as a CPU or a processor, reading and executing a software program stored in a recording medium, such as a hard disk or semiconductor memory.

Moreover, the order in which the steps in each flowchart are performed is provided as an example to specifically explain the present disclosure. The steps may be performed in order different from the above order. For instance, the processing in step S14 indicated in FIG. 8 may be performed between steps S12 and S13. When the determination result is No in step S14, since external light is entering the oral cavity, the dental plaque detection processing in step S13 may be omitted. Moreover, one or more of the above steps may be performed concurrently (in parallel) with another step, and one or more of the above steps need not be performed.

Moreover, the allocation of the functional blocks illustrated in each block diagram is a mere example. Two or more functional blocks may be incorporated into one functional block. One functional block may be divided into more than one functional block. A part of the function may be transferred from one functional block to another functional block. Moreover, the same hardware or software may process the functions of two or more functional blocks having similar functions in parallel or on a time-sharing basis.

Moreover, portable terminal 50 according to the embodiment and the variations thereof may be achieved as a single device or a plurality of devices. When portable terminal 50 is achieved as a plurality of devices, the constituent elements of portable terminal 50 may be divided into the devices in what ways. For instance, among the functions of portable terminal 50, at least one or more functions may be achieved by intraoral camera 10 (for example, signal processor 30). When portable terminal 50 is achieved as devices, a communication method between the devices is not limited to a particular method, and the communication may be wireless or wired communication. Moreover, a combination of wireless communication and wired communication may be used as communication between devices.

Moreover, each of the constituent elements described in the embodiment and the variation thereof may be achieved as software and may be achieved typically as LSI, which is an integrated circuit. Those elements may be made as individual chips. Some or all of the elements may be incorporated into one chip. Although the term LSI is used here, the terms IC, system LSI, super LSI, and ultra LSI may be used according to the difference in the integration degree. Moreover, the method of circuit integration is not limited to an LSI, and circuit integration may be achieved as a dedicated circuit (a general-purpose circuit for executing a dedicated program) or a general-purpose processor. A field programmable gate array (FPGA), which can be programmed after manufacturing an LSI, or a reconfigurable processor in which the connections or settings of circuit cells inside an LSI are reconfigurable may be used. Furthermore, if a circuit integration technology that will replace an LSI appears with the development of the semiconductor technology or another derivative technology, constituent elements may be of course integrated using the new technology.

A system LSI is a super multifunctional LSI manufactured by integrating a plurality of processing units into one chip, and specifically a computer system including a microprocessor, ROM, and RAM. A computer program is stored in ROM. The system LSI performs its function by the microprocessor operating in accordance with the computer program.

Moreover, another aspect of the present disclosure may be a computer program for causing a computer to execute the characteristic steps included in the image processing method illustrated in FIG. 8 or 14.

Moreover, for example, the program may be a program to be executed by the computer. Moreover, still another aspect of the present disclosure may be a non-transitory computer-readable recording medium in which such a program is stored. For instance, the program recorded on the recording medium may be distributed. For instance, by installing the distributed program onto a device including another processor, and causing the processor to execute the program, it is possible to make the device perform each processing task described above.

### [Industrial Applicability]

The present disclosure is applicable to intraoral camera systems.

### [Reference Signs List]

- 10: intraoral camera
- 10a: head
- 10b: handle
- 10c: neck
- 20: hardware
- 21: imaging unit
- 22: sensor
- 23: illuminator
- 23A: first LED
- 23B: second LED
- 23C: third LED
- 23D: fourth LED
- 24: operation interface
- 30: signal processor
- 31: camera controller
- 32: image processor
- 33, 52: controller
- 34: illumination controller
- 35: memory
- 40: communicator
- 50: portable terminal
- 51: obtainer
- 53: display controller
- 54: touch screen
- R1, R2, R3, R4, R11, R12, R13, R14, R21, R22, R23, R24, R25, R26, R27, R28, R29: area

## Claims

1. An image processing method for displaying a dental plaque area based on an RGB image obtained by capturing an image of a tooth and dental plaque fluorescing in response to exposure to light including a blue-light wavelength range, the image processing method comprising:
obtaining an incident level of external light entering an imaging area of a first RGB image;
performing dental plaque detection processing based on the first RGB image; and
changing a display mode of a dental plaque detection result according to the incident level of the external light which has entered the imaging area of the first RGB image.

2. The image processing method according to claim 1, comprising:
when the incident level is not lower than or equal to a predetermined value, displaying an image in which information indicating that the external light is entering is superposed onto an image displaying the dental plaque detection result.

3. The image processing method according to claim 2, comprising:
when the incident level is lower than or equal to the predetermined value, displaying an image in which information indicating that the external light is not entering is superposed onto an image displaying the dental plaque detection result.

4. The image processing method according to claim 1, comprising:
when the incident level is not lower than or equal to a predetermined value, prohibiting displaying of the dental plaque detection result.

5. The image processing method according to any one of claims 1 to 4, wherein
the incident level is obtained based on a detection result of a detector that detects the external light entering the imaging area of the first RGB image.

6. The image processing method according to any one of claims 1 to 4, wherein
the incident level is calculated based on a first red pixel count indicating a total number of red pixels having red pixel values greater than or equal to a first pixel value in the first RGB image.

7. The image processing method according to claim 6, wherein
the incident level includes a first incident level,
the first red pixel count includes, for each of pixel values greater than or equal to the first pixel value, a first pixel count indicating a total number of red pixels having the pixel value, and
a green pixel count indicating a total number of green pixels having green pixel values greater than or equal to the first pixel value includes, for each of the pixel values greater than or equal to the first pixel value, a second pixel count indicating a total number of green pixels having the pixel value,
the image processing method comprising:
subtracting the second pixel count from the first pixel count in each of the pixel values greater than or equal to the first pixel value;
calculating the first incident level in accordance with a first integrated value calculated by integrating one or more positive first subtraction values among first subtraction values each of which is calculated by subtracting the second pixel count from the first pixel count; and
changing the display mode of the dental plaque detection result according to whether the first incident level is lower than or equal to a first predetermined value.

8. The image processing method according to claim 6, wherein
the incident level includes a first incident level,
the first red pixel count includes a first pixel count indicating a total number of red pixels having pixel values greater than or equal to the first pixel value, and
a green pixel count indicating a total number of green pixels having green pixel values less than or equal to a fourth pixel value lower than the first pixel value includes a second pixel count indicating a total number of green pixels having pixel values less than or equal to the fourth pixel value,
the image processing method comprising:
subtracting the second pixel count from the first pixel count;
when a first subtraction value calculated by subtracting the second pixel count from the first pixel count is a positive value, calculating the first incident level in accordance with the first subtraction value; and
changing the display mode of the dental plaque detection result according to whether the first incident level is lower than or equal to a first predetermined value.

9. The image processing method according to claim 7, wherein
the incident level includes a second incident level, and
a second red pixel count indicating a total number of red pixels having red pixel values greater than or equal to a second pixel value higher than the first pixel value includes, for each of pixel values greater than or equal to the second pixel value, a third pixel count indicating a total number of red pixels having the pixel value,
the image processing method comprising:
subtracting the second pixel count from the third pixel count in each of the pixel values greater than or equal to the second pixel value;
calculating the second incident level in accordance with a second integrated value calculated by integrating one or more positive second subtraction values among second subtraction values each of which is calculated by subtracting the second pixel count from the third pixel count; and
changing the display mode of the dental plaque detection result according to whether the second incident level is lower than or equal to a second predetermined value.

10. The image processing method according to claim 7, wherein
the incident level includes a third incident level,
the image processing method comprising:
generating a V image having V values obtained by performing HSV conversion on pixel values of the first RGB image;
calculating the third incident level in accordance with an integrated value indicating a total number of pixels whose V values are greater than or equal to a third pixel value; and
changing the display mode of the dental plaque detection result further according to whether the third incident level is lower than or equal to a third predetermined value.

11. The image processing method according to claim 10, comprising:
when the first incident level is at most the first predetermined value and at least a fourth predetermined value lower than the first predetermined value and when the third incident level is at most the third predetermined value and at least a fifth predetermined value lower than the third predetermined value,
displaying an image in which information suggesting blocking light is superposed onto an image displaying the dental plaque detection result.

12. The image processing method according to claim 6, comprising:
generating a V image having V values obtained by performing HSV conversion on pixel values of the first RGB image; and
calculating the incident level in accordance with an integrated value indicating a total number of pixels whose V values are greater than or equal to the first pixel value.

13. The image processing method according to any one of claims 2 to 4, wherein
the predetermined value is set according to a lighting environment of a space where the first RGB image is to be captured.

14. The image processing method according to any one of claims 2 to 4, comprising:
dividing the first RGB image into a plurality of areas;
obtaining the incident level of the external light entering each of the plurality of areas into which the first RGB image has been divided; and
changing the display mode of the dental plaque detection result according to a result of determination as to whether the incident level in each of the plurality of areas is lower than or equal to the predetermined value.

15. The image processing method according to any one of claims 2 to 4, comprising:
displaying solution information that depends on the incident level to suppress an effect of the external light.

16. The image processing method according to claim 15, comprising:
dividing the first RGB image into a plurality of areas;
obtaining the incident level of the external light entering each of the plurality of areas into which the first RGB image has been divided; and
displaying solution information that depends on at least one of a total number of one or more areas, among the plurality of areas, in each of which the incident level exceeds the predetermined value or positions of the one or more areas among the plurality of areas.

17. The image processing method according to claim 16, comprising:
a determiner that determines whether a user has responded to the solution information;
after the user is determined to have responded to the solution information, obtaining post-response incident levels in the plurality of areas in a second RGB image obtained by capturing an image of the tooth and the dental plaque fluorescing in response to exposure to the light including the blue-light wavelength range; and
displaying different solution information based on the post-response incident levels in the plurality of areas, the different solution information being information different from the solution information.

18. The image processing method according to claim 17, wherein
the different solution information is information that depends on at least one of a total number of one or more areas, among the plurality of areas, in each of which a post-response incident level exceeds the predetermined value or positions of the one or more areas among the plurality of areas.

19. The image processing method according to claim 16, comprising:
determining an incident direction of the external light in accordance with positions of the one or more areas in each of which the incident level exceeds the predetermined value among the plurality of areas, wherein
the solution information includes information on the incident direction of the external light.

20. An image processing system for displaying a dental plaque area based on an RGB image obtained by capturing an image of a tooth and dental plaque fluorescing in response to exposure to light including a blue-light wavelength range, the image processing system comprising:
an obtainer that obtains an incident level of external light entering an imaging area of the RGB image;
a dental plaque detector that performs dental plaque detection processing based on the RGB image; and
a display controller that changes a display mode of a dental plaque detection result according to the incident level of the external light which has entered the imaging area of the RGB image.

21. A program for causing a computer to execute the image processing method according to any one of claims 1 to 4.
